(19) **Europäisches Patentamt / European Patent Office / Office européen des brevets**

(11) **EP 4 759 288 A1**

(12) **EUROPEAN PATENT APPLICATION**
published in accordance with Art. 153(4) EPC

(43) Date of publication:
**17.06.2026 Bulletin 2026/25**

(21) Application number: **25783097.6**

(22) Date of filing: **03.02.2025**

(51) International Patent Classification (IPC):
**A61H 1/02** (2006.01)   **A61B 5/11** (2006.01)
**G16H 10/60** (2018.01)   **G16H 20/30** (2018.01)
**G16H 50/20** (2018.01)

(52) Cooperative Patent Classification (CPC):
**A61B 5/11; A61H 1/02; G16H 10/60; G16H 20/30; G16H 50/20**

(86) International application number:
**PCT/KR2025/099186**

(87) International publication number:
**WO 2025/211921 (09.10.2025 Gazette 2025/41)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.04.2024  KR 20240044326**
**21.08.2024  KR 20240112332**
**11.10.2024  KR 20240138425**
**17.12.2024  KR 20240188864**
**20.01.2025  KR 20250007941**

(71) Applicant: **Curexo, Inc.**
**Seoul 05814 (KR)**

(72) Inventors:
• **HWANG, Sun Hee**
**Sokcho-si, Gangwon-do 24809 (KR)**
• **LEE, Sang Hun**
**Seoul 07064 (KR)**
• **KIM, Young Hwan**
**Osan-si, Gyeonggi-do 18132 (KR)**
• **MOON, Jun Sik**
**Seoul 02716 (KR)**

(74) Representative: **Lavoix**
**Bayerstraße 83**
**80335 München (DE)**

(54) **ARTIFICIAL INTELLIGENCE-BASED ROBOT-ASSISTED ORTHOPEDIC EXERCISE SYSTEM AND METHOD**

(57)   Provided is an exercise system including: a gait training apparatus including a pedal on which a patient stands and a pedal driver providing one or more links that drive the pedal; and a system control unit configured to perform gait training of the patient according to a patient-tailored training plan, wherein the system control unit includes: an index prediction unit configured to determine a gait evaluation index of the patient, based on basic information including patient's personal information and initial gait status information; a training planning unit including an autonomous training planning model configured to establish, based on a result of the gait training, the patient-tailored training plan performed by the gait training apparatus; and a training evaluation unit configured to evaluate a training result, based on training result data according to results of rehabilitation training of the patient performed by the gait training apparatus.

FIG. 9

| SYSTEM CONTROL UNIT | 111 |
| INPUT UNIT | 111a |
| INDEX PREDICTION UNIT (M1) | 111b |
| TRAINING PLANNING UNIT (M2) | 111c |
| TRAINING PROGRESS UNIT (M3) | 111d |
| TRAINING EVALUATION UNIT (M4) | 111e |
| REPORTING UNIT (M5) | 111f |

EP 4 759 288 A1

## Description

### Technical Field

The present disclosure relates to a robot-assisted orthopedic exercise system, and more particularly, to a patient-friendly artificial intelligence (AI)-based assisted orthopedic exercise system and a method of controlling the same, in which an individual-tailored orthopedic exercise plan may be automatically established and executed and the exercise plan may be automatically corrected according to a rehabilitation effect of the exercise, based on AI.

### Background Art

Robot-assisted orthopedic exercise systems have been developed as devices for helping the gait rehabilitation of patients having walking difficulties. Such a system improves the gait function of patients having walking difficulties and helps restore the muscular strength of the lower body and includes a so-called end-effector-type robot device to support and assist the physical movement of a patient and aid the patient with further safe and effective gait rehabilitation.
The system may be adjusted according to the physical ability and status of the patient, may provide gait training via simulation or assistance of gait motions, and may sense and correct motions of the patient, thereby further improving the gait abilities safely and efficiently and helping recovering independence in daily life.
According to such a previous system, a therapist accompanies patients to guide and assist them in a rehabilitation exercise process. However, depending on cases, some patients cannot adapt to the operation of pedals and fail to adapt to or comply with motions of the pedals. Also, there are cases in which effective rehabilitation is hard to achieve even though gait training is performed for a long time.
Therefore, the development of a rehabilitation training system for performing safe and effective rehabilitation training so as to enable patients to quickly rehabilitate and get back to a normal life would be highly helpful.

### Disclosure of Invention

### Technical Problem

The present disclosure provides an artificial intelligence (AI)-based robot-assisted orthopedic exercise system and method capable of establishing patient-friendly gait training plans.
The present disclosure provides an AI-based robot-assisted orthopedic exercise system and method capable of reducing intervention of a therapist according to the prescription of medical staff.
The present disclosure provides a patient-personalized and friendly AI-based robot-assisted orthopedic exercise system and method.

### Solution to Problem

An artificial intelligence (AI)-based robot-assisted orthopedic exercise system according to the present disclosure includes:

a gait training apparatus including a pedal on which a patient stands and a pedal driver providing one or more links that drive the pedal; and a system control unit configured to control the pedal driver according to a training plan established for each individual to perform gait training of the patient standing on the pedal,
wherein the system control unit includes:

an index prediction unit configured to determine a gait evaluation index of the patient, based on patient's initial gait status information or information about a gait training result after initial gait training;
a training planning unit configured to establish, based on the gait training result, an individual-tailored training plan of the patient, performed by the gait training apparatus;
a training progress unit configured to perform training on the patient according to the training plan, monitor, in real time, training data with respect to a real-time training status from the patient, and generate feedback with respect to the training data; and
a training evaluation unit including a training evaluation model configured to evaluate a training result, based on training result data according to results of rehabilitation training of the patient performed by the gait training apparatus.

According to an embodiment, the training data may include information about at least one characteristic from among a ground reaction force (GRF) (%), a body weight support (BWS) (%), an upper body inclination or tilting (degree), a joint angle (degree), a cadence, a left gait cycle (%), a right gait cycle (%), a stride length, an ankle angle, oxygen saturation data, and heart rate data.

According to an embodiment, a training evaluation unit may include a training evaluation model configured to evaluate a training result, based on training result data according to results of rehabilitation training of the patient performed by the gait training apparatus,

the training evaluation model may be configured to provide an anomaly detection model and a trend prediction model, and

the anomaly detection model may detect abnormal patterns in real-time training data, and the trend prediction model may predict future training performance based on past training data and current training data.

According to an embodiment, the anomaly detection model may detect the abnormal patterns in the real-time training data, based on at least one of a long short-term memory (LSTM) autoencoder or a variational autoencoder (VAE), and

the trend prediction model may predict the future training performance, based on past data and current data, by utilizing a Seq2Seq-based LSTM/gated recurrent unit (GRU) or transformer-based model.

According to an embodiment, the training progress unit may include a training progress model configured to generate the feedback on the training data, and

the training progress model may be configured to receive at least one training parameter from among a step length (cm), a step height (cm), an initial contact angle (degree), and a toe off angle (degree) and output, based on the training data, a correction value for the at least one training parameter.

According to an embodiment, the training progress model may include any one deep learning model from a multi-layer perceptron (MLP) and an open vision-language model (VLM).

According to an embodiment, image information or voice information of the patient undergoing training or a therapist assisting the patient's training may be obtained and used as input data of the training progress model, and the training progress model may be configured to generate output data corresponding to the input data.

According to an embodiment, the training planning unit may further include a reference training planning model trained using patient information, training protocols, training time, training parameters, etc. set by a medical professional, and

the training planning unit may be configured to output a final training plan that is corrected or compensated based on outputs of an autonomous training planning model and the reference training planning model.

According to an embodiment, the training planning unit may further include a final training planning model configured to output the final training plan based on the outputs of the autonomous training planning model and the reference training planning model.

According to an embodiment, the AI-based robot-assisted orthopedic exercise system may further include a monitoring unit configured to monitor a status of the rehabilitation training performed by the gait training apparatus according to the training plan, obtain information during the training, and generate therefrom training status information with respect to the patient's gait training.

According to one embodiment, the monitoring unit may be configured to provide, to the patient, training guidelines or recommended guidelines for gait improvement with respect to the gait training or a current training state, based on the information during the training.

A method of providing an artificial intelligence (AI)-based robot-assisted orthopedic exercise according to the present disclosure includes:

predicting, by an index prediction unit, a gait evaluation index of a patient, based on initial gait status information or information about a gait training result after initial gait training;

establishing, by a training planning unit, an individual-tailored training plan of the patient, by using an autonomous training planning model based on the gait evaluation index;

while a gait training apparatus including an end-effector-type pedal and a pedal driver providing one or more links that drive the pedal, is performing rehabilitation training of the patient according to the training plan, monitoring, by a training progress unit, in real time, training data with respect to a real-time training status from the patient and generating feedback with respect to the training data; and

evaluating, by a training evaluation unit, the training results, based on training result data according to training results.

In the method of providing the AI-based robot-assisted orthopedic exercise according to an embodiment, the training data may include at least one from among a ground reaction force (GRF), a body weight support (BWS), an upper body inclination or tilting, a joint angle, a cadence, a left gait cycle, a right gait cycle, a stride length, an ankle angle, oxygen saturation data, and heart rate data.

In the method of providing the AI-based robot-assisted orthopedic exercise according to an embodiment, the training evaluation unit may include a training evaluation model configured to evaluate the training result, based on the training result data based on results of the rehabilitation training of the patient performed by the gait training apparatus,

the training evaluation model may be configured to provide an anomaly detection model and a trend prediction model, and

the anomaly detection model may detect abnormal patterns in real-time training data, and the trend prediction model may predict future training performance based on past training data and current training data.

In the method of providing the AI-based robot-assisted orthopedic exercise according to an embodiment, the anomaly detection model may detect the abnormal patterns in the real-time training data, based on at least one of a long short-term memory (LSTM) autoencoder or a variational autoencoder (VAE), and

the trend prediction model may predict the future training performance, based on past data and current data, by utilizing a Seq2Seq-based LSTM/gated recurrent unit (GRU) or transformer-based model.

In the method of providing the AI-based robot-assisted orthopedic exercise according to an embodiment, the training progress unit may include a training progress model configured to generate the feedback on the training data, and the training progress model may be configured to receive at least one training parameter from among a step length (cm), a step height (cm), an initial contact angle (degree), and a toe off angle (degree) and output, based on the training data, a correction value for the at least one training parameter.

In the method of providing the AI-based robot-assisted orthopedic exercise according to an embodiment, the training progress model may include any one deep learning model from a multi-layer perceptron (MLP) and an open vision-language model (VLM).

In the method of providing the AI-based robot-assisted orthopedic exercise according to an embodiment,

an image or voice recording device may be configured to obtain image information or voice information of the patient undergoing training or a therapist assisting the patient's training,

an analysis device may be configured to analyze the image information or the voice information, and

the training progress model may be configured to use input data of the training progress model and generate output data corresponding to the input data.

In the method of providing the AI-based robot-assisted orthopedic exercise according to an embodiment, the training planning unit may further include:

the autonomous training planning model that establishes the individual-tailored training plan of the trained patient according to the initial gait status information or the gait training result after the gait training; and

a reference training planning model trained using patient information, training protocols, training time, training parameters, etc. set by a medical professional, and

the training planning unit may be configured to output a final training plan that is corrected or compensated based on outputs of the autonomous training planning model and the reference training planning model.

In the method of providing the AI-based robot-assisted orthopedic exercise according to an embodiment, a training-status detection unit may detect, as characteristics information during training, information about at least one of a GRF, a BWS, an upper body inclination or tilting, a joint angle, a cadence, a left gait cycle, a right gait cycle, a stride length, an ankle angle, oxygen saturation data, and heart rate data of the patient undergoing the gait training.

A monitoring unit may be configured to monitor a status of the rehabilitation training performed by the gait training apparatus according to the training plan, obtain information during the training, and generate therefrom training status information with respect to the patient's gait training.

In the method of providing the AI-based robot-assisted orthopedic exercise according to an embodiment, a reporting unit may be configured to analyze previous training results and current training results to generate reporting content in the form of text, images, and videos.

In the method of providing the AI-based robot-assisted orthopedic exercise according to an embodiment, a head-mounted display (HMD) may present necessary information related to a gait exercise of the patient, training status information, or recommended guidelines related to training to the patient.

**Advantageous Effects of Invention**

According to the present disclosure, a patient-friendly gait training plan may be established, intervention of a therapist may be reduced compared to the related art, and especially, patient-personalized and friendly rehabilitation training may be efficiently performed. According to rehabilitation training according to a system and method as such, gait evaluation indices may be accurately predicted using an artificial intelligence (AI) model at each operation, a training plan may be automatically established based on the accurate prediction, and training results may be further accurately evaluated.

**Brief Description of Drawings**

FIG. 1 is a block diagram of an artificial intelligence (AI)-based robot-assisted orthopedic exercise system according to the present disclosure,

FIG. 2 is a schematic perspective view of an AI-based robot-assisted orthopedic exercise system according to an embodiment of the present disclosure,

FIG. 3 is a lateral view of an AI-based robot-assisted orthopedic exercise system according to an embodiment of the present disclosure,

FIG. 4 is a front view of an AI-based robot-assisted orthopedic exercise system according to an embodiment of the present disclosure,

FIG. 5 illustrates a scene in which a patient is training while wearing a head-mounted display (HMD) in a gait training system according to the present disclosure, and

FIG. 6 is a schematic perspective view of a gait training apparatus of an AI-based robot-assisted gait training system according to the present disclosure.

FIG. 7 illustrates a normal gait cycle and postures of feet, knees, and thighs during a normal gait,

FIG. 8 illustrates regulations at each position of a gait pattern,

FIG. 9 illustrates functional parts based on software and hardware structures of a system control device according to the present disclosure,

FIG. 10 illustrates a structure of a data set used in a gait evaluation indices prediction model implemented in an AI-based robot-assisted orthopedic exercise method and system according to an embodiment of the present disclosure,

FIG. 11 is a flowchart of a training process for a new patient in an AI-based robot-assisted orthopedic exercise provision method according to an embodiment of the present disclosure,

FIG. 12 is an overall flowchart of an AI-based robot-assisted orthopedic exercise provision method according to an embodiment of the present disclosure,

FIG. 13 illustrates a code snippet with respect to a class of a long short-term memory (LSTM) model in an AI-based robot-assisted orthopedic exercise method and system according to an embodiment of the present disclosure,

FIG. 14 illustrates a code snippet for defining a forward propagation process of a model in an AI-based robot-assisted orthopedic exercise method and system according to an embodiment of the present disclosure,

FIG. 15 illustrates a code snippet for a definition of a Python class implementing early stopping in an AI-based robot-assisted orthopedic exercise method and system according to an embodiment of the present disclosure,

FIG. 16 illustrates a code snippet for a definition of a train_model, a function for model training in an AI-based robot-assisted orthopedic exercise method and system according to an embodiment of the present disclosure,

FIG. 17 illustrates a code snippet for a data evaluation function for predicting functional ambulation categories (FAC) of a patient by evaluating new data from a corresponding gait-training patient in an AI-based robot-assisted orthopedic exercise method and system according to an embodiment of the present disclosure,

FIG. 18 illustrates a code snippet with respect to execution of an LSTM model in an AI-based robot-assisted orthopedic exercise method and system according to an embodiment of the present disclosure,

FIG. 19 illustrates a code snippet with respect to data inputs for training a model and generation of a data frame in which the data inputs are accumulated, according to an embodiment of the present disclosure,

FIG. 20 illustrates a code snippet with respect to declaration of a transformer model, training setting, model generation, etc., according to an embodiment of the present disclosure,

FIG. 21 illustrates a code snippet with respect to training of a transformer model and prediction of test data, according to an embodiment of the present disclosure,

FIG. 22 illustrates a graphics interface showing various parameters of gait training according to an embodiment of the present disclosure, and

FIG. 23 is a butterfly diagram showing average plantar pressure distribution of a patient during training, as a result of gait training of a patient,

FIG. 24 illustrates a code snippet of a declaration unit of a data set class for training of Model 3 according to an embodiment of the present disclosure,

FIG. 25 illustrates a code snippet for defining Model 3 according to an embodiment of the present disclosure,

FIG. 26 illustrates a code snippet of a feedback generation function for outputting feedback provided to a patient, according to an embodiment of the present disclosure,

FIG. 27 illustrates a code snippet of a real-time data processing function according to an embodiment of the present disclosure,

FIG. 28 illustrates a code snippet of a function for training of Model 3, according to an embodiment of the present disclosure,

FIG. 29 illustrates a code snippet of a function for evaluating trained Model 3, according to an embodiment of the present disclosure, and

FIG. 30 illustrates a snippet of an execution code for generating trained Model 3, according to an embodiment of the present disclosure.

FIG. 31 illustrates a code snippet of a data pre-processing and training pipeline using an LSTM model for anomaly detection and trend prediction, according to the present disclosure.

FIG. 32 illustrates a code snippet showing declaration of an LSTM-based auto encoder class and a Seq2Seq class for anomaly detection and trend prediction and an initialization process of two models using the classes, according to the present disclosure.

FIG. 33 illustrates a definition of a class of a function <train_anomaly_model> for training of an anomaly detection model M4a, which is an element of a M4 model, according to the present disclosure.

FIG. 34 illustrates a definition of a function for training of a trend prediction model M4b of an M4 model, according to the present disclosure.

FIG. 35 illustrates a definition of a function, evaluate_anomaly_model, for evaluating an anomaly detection model M4a in a M4 model, according to the present disclosure.

FIG. 36 illustrates a pseudo code snippet of a function for visualizing and analyzing a test result and presenting an improvement suggestion, in an M4 model, according to the present disclosure.

## Mode for the Invention

Hereinafter, preferred embodiments of the present inventive concept are described in detail with reference to the accompanying drawings. However, the embodiments of the present inventive concept may be modified into various other forms, and the scope of the present inventive concept shall not be construed as being limited to the embodiments described below in detail. It is desirable that the embodiments of the present inventive concept be interpreted as being provided to more fully explain the present inventive concept to one of ordinary skill in the art. The same signs denote the same elements throughout. Furthermore, various elements and areas in the drawings are schematically drawn. Therefore, the present inventive concept is not limited by the relative sizes or distances illustrated in the accompanying drawings.

Terms such as first, second, etc. may be used to describe various components, but the components are not defined by these terms. The terms are used only for distinguishing one element from another element. For example, without deviating from the scope of the claims of the present inventive concept, a first element may be referred to as a second element, and vice versa.

The terms used in the present application are used only for describing particular embodiments and are not intended to limit the present inventive concept. A singular expression may include a plural expression, unless an apparently different meaning is indicated in the context. With respect to the present application, it will be further understood that the expressions "comprises" and "comprising" used herein specify the presence of stated features, integers, steps, operations, members, components, and/or groups thereof, but do not preclude the presence or addition of one or more other features, integers, operations, members, components, and/or groups thereof.

Unless otherwise defined, all of the terms used herein including technical terms and scientific terms have the same meaning as the meaning commonly understood by one of ordinary skill in the art of the present inventive concept. Also, the terms commonly used and having the meanings as defined in dictionaries shall be understood to have consistent meanings with the corresponding terms in the context of relevant arts, and unless explicitly defined so herein, the meanings of the terms shall not be understood to be excessively formal.

When a certain embodiment may be implemented differently, a specific process order may be performed differently from the described order. For example, two consecutively described processes may be performed substantially at the same time or performed in an order opposite to the described order.

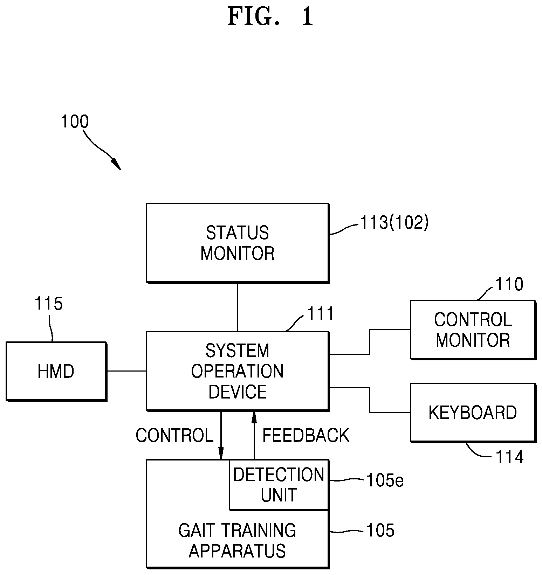

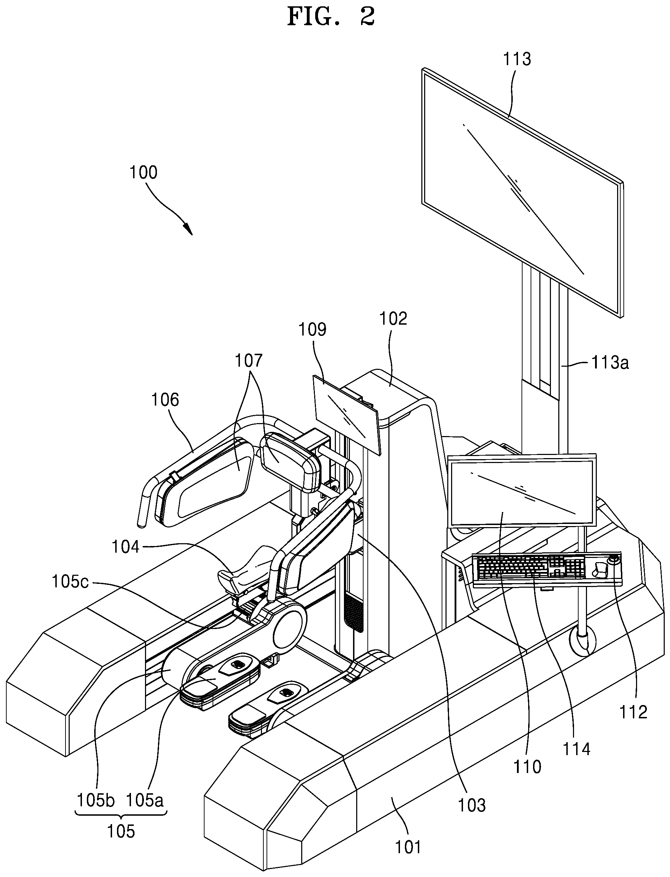

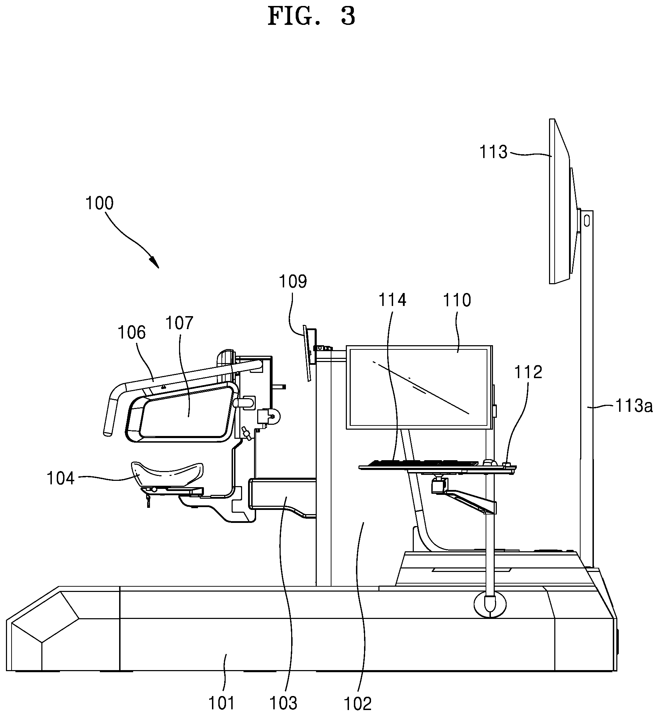

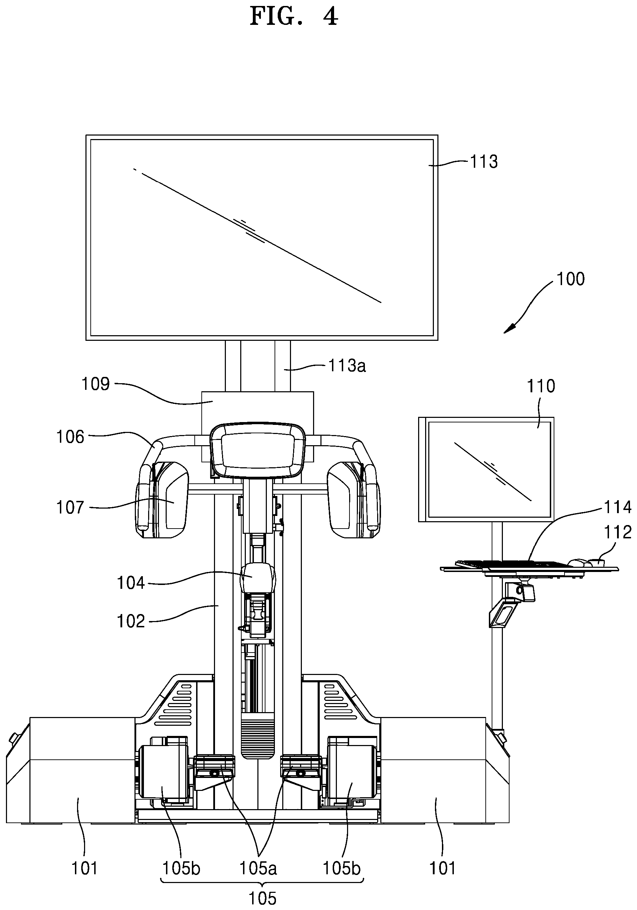

FIG. 1 is a block diagram of an artificial intelligence (AI)-based robot-assisted orthopedic exercise system according to the present disclosure, FIG. 2 illustrates an embodiment of the AI-based robot-assisted orthopedic exercise system according to the present disclosure, and FIGS. 3 and 4 are a lateral view and a front view, respectively, of the same.

First, referring to FIG. 1, an exercise system 100 may include a system control unit 111 based on a complex structure including hardware and software, a gait training apparatus 105 controlled by the system control unit 111, a main or control monitor 110 configured to control the system control unit 111, a status display monitor 113 configured to display a patient's exercise status, etc., a complex input unit 114 including a keyboard 112 and configured to input various electrical and optical data, and a head-mounted display (HMD) 115 as an additional option, which is worn by a patient. The system control unit 111 according to the present disclosure may have a structure of a computer system based on a virtual agent including one or more models trained through machine learning or deep learning and configured to perform data processing and prediction.

The gait training apparatus 105 may include two pedals on the right and left sides, on which a patient stand, and driving devices on the right and left sides which are configured to drive the two pedals, and a training-status detection unit 105e having various sensors configured to detect patient's status information during gait training may be provided directly in or around the gait training apparatus 105.

The training-status detection unit 105e may include not only a sensor configured to detect a signal, but also a signal processor configured to generate a signal to be transmitted to the system control unit 111. The HMD 115 may be configured to present necessary information to a patient during training and may be part of a motor device in the exercise system 100.

Additionally, the system control unit 111 may further include an optical detection device configured to detect a patient's exercise status, etc., for example, a video camera, such as a webcam, that is an optical input device.

Referring to FIGS. 2 to 4, the exercise system 100 may be a type of robot providing gait training to a patient and may provide two right and left gait training apparatuses 105 controlled by the system control unit 111. The two gait training apparatuses 105 may be arranged between a left main body base 101 and a right main body base 101 arranged on left and right sides in parallel to be apart from each other by a certain distance. The two pedals 105a of the two right and left gait training apparatuses 105 may harmoniously move according to a movement trajectory of three degrees of freedom, such as rotations of both right and left ankles, top and down and forward and backward movements of both right and left feet, etc., during a gait, and in some cases, may intentionally inharmoniously move.

The pedals may perform motions to allow the patient to perform an appropriate gait exercise according to a control signal from the system control unit 111, and the patient may stand on the pedals and perform a gait exercise in compliance with a training gait trajectory determined by the motions of the pedals. An ankle band which may be mounted in this case may fix a foot of the patient appropriately loosely or appropriately tightly to the pedal by a degree in which the foot on the pedal may move within a certain range.

The motions of the two pedals 105a may be configured for the gait training of the patient and may be controlled by the system control unit to accept an abnormal gait pattern and to be trained to make the gait pattern close to a normal gait pattern.

For this gait training, the gait training apparatus 105 at the left side may include the pedal 105a and an operating link 105b to which the pedal 105a is dynamically linked, and the operating link 105b of the gait training apparatus at the left side may be coupled, through the medium of a first driving device 105c provided in the left main body base 101, to the left main body base 101 to be capable of a linear reciprocating motion and a rotary motion, and the gait training apparatus 105 at the right side may also be coupled, through the medium of the first driving device 105c of the right main body base 101, to the right main body base 101 to be capable of a reciprocating motion and a rotary motion. The pedal 105a may be coupled to the operating link 105b through a driving device, for example, a driving device, a rotational angle of which may be controlled, and the pedal 105a may perform a relative motion forcibly by the driving device within a certain angular range with respect to the operating link 105b.

The linear reciprocating motion of each of the gait training apparatuses 105 through the first driving device may be separately performed, but they may get further apart from each other or get closer to each other and overlap each other according to the motions of the both right and left feed during a gait. The first driving device 105c may include a plurality of driving devices configured to cause the linear reciprocating motion and the rotary motion of the operating link 105b. For example, the first driving device 105c may have a combination of a linear motion driving device for controlling linear reciprocating motions of the operating link or the entire gait training apparatus 105 including the operating link and a rotational or pivotal motion driving device for controlling a rotary motion of the operating link 105b.

In front of the gait training apparatus 105, an assembly combining a saddle 104, a safety rod 106 thereabove or a fence portion 107 including the safety rod 106 and supporting the chest portion, etc. are provided. The assembly may be mounted on a lift device 102 through the medium of a top and bottom frame 103. The lift device 102 may include an assembly ascending and descending device configured to adjust the height of the assembly to correspond to a physical condition of a patient. A system status display 109 may be provided above the lift device 102 to face the patient.

An ascending and descending-type or a fixed-type support column 113a may be mounted on the foremost end of the system 100 toward which the patient is positioned, and a status display monitor 113 configured to display the operating status of the entire system may be coupled to the support column 113a.

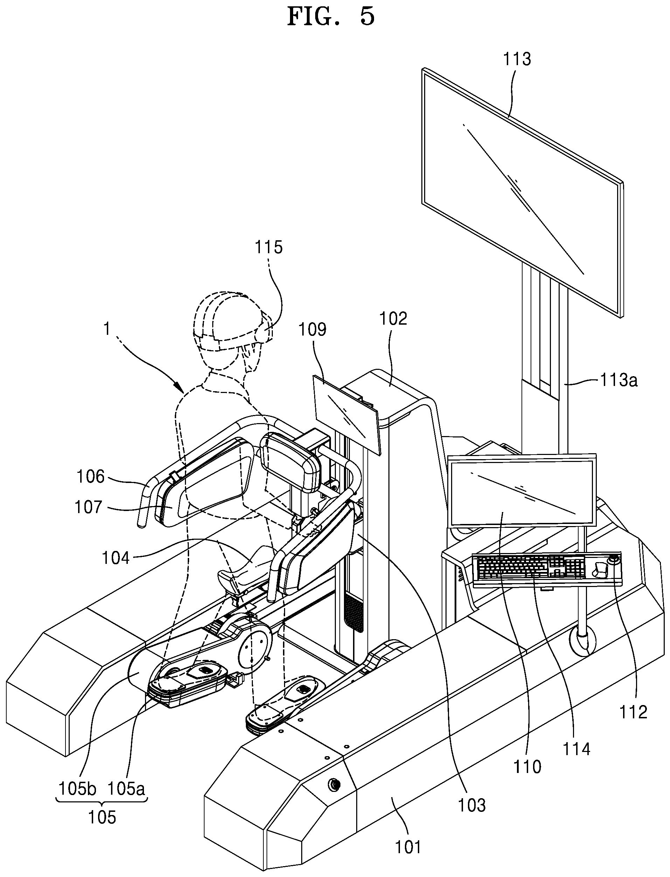

FIG. 5 illustrates a scene in which a patient 1 is trained while wearing the HMD 115 to be described below, in the gait training system 100 according to the present disclosure. As illustrated in FIG. 5, for example, in the case of a severe patient, the patient 1 may perform gait training while grabbing the safety rod 106 while sitting on the saddle 104. Depending on the condition of the patient 1, for example, in the case of a patient having light symptoms, the saddle 104 may be folded, so that the patient 1 may perform gait training without depending on the saddle 104.

The pedal 105a may be operated by the system control unit to force a gait of the patient regardless of the will of the patient. Here, a pressure sensor, etc. may be mounted on the saddle 104 and the pedal 105a to detect the load applied to the saddle 104 and the load applied to the pedal during the condition of the gait training. In particular, a plurality of pressure sensors may be provided in front of and behind the pedals to detect the local pressure applied to the pedals, and thus, the degree of pressurization of the sole of the foot or the contact of the sole of the foot with respect to the pedal may be detected.

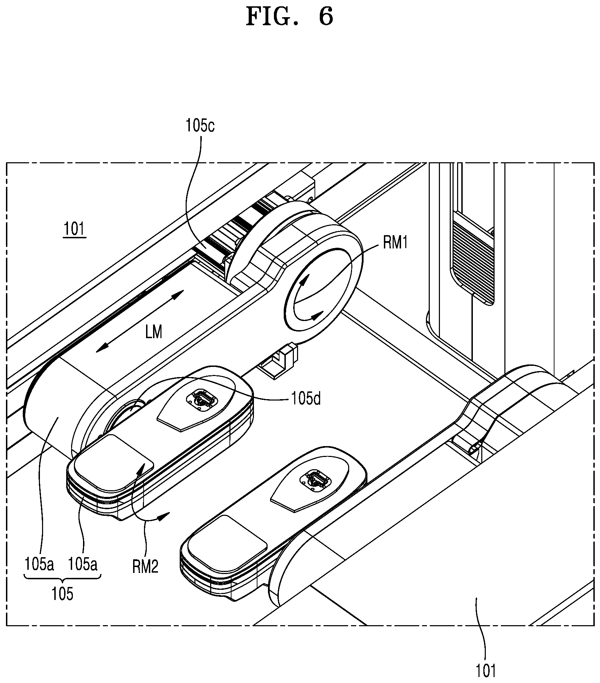

FIG. 6 is a schematic perspective view of the gait training apparatus 105 dynamically coupled to the main body base 101.

Referring to FIG. 6, the operating link 105b may be coupled to the main body base 101 through the medium of the first driving device 105c, to be capable of linear reciprocating movement LM and rotational movement RM1. According to an embodiment, the first driving, 105c may include a driving motor and an additional reciprocating movement driving device may be connected to a side of or below the first driving device 105c. Also, the pedal 105a may be coupled, through the medium of a second driving device 105d, to the operating link 105b to be capable of rotational movement RM2 by a certain angle, and here, a band may be mounted to bind the patient's foot or ankle.

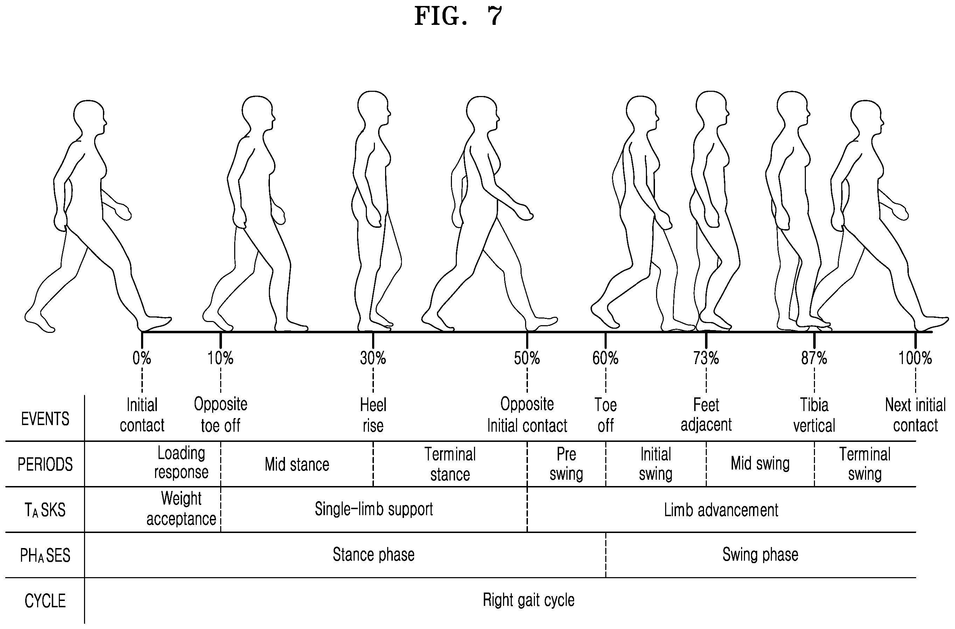

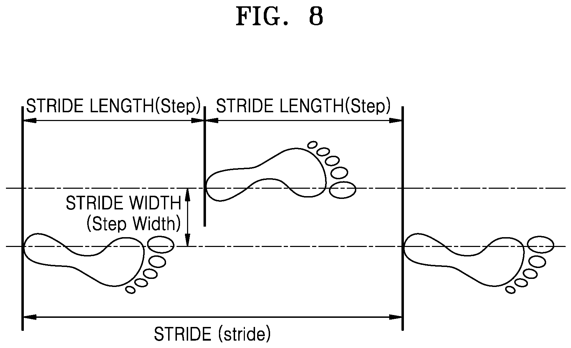

FIG. 7 illustrates a normal gait cycle, showing the postures of the feet, knees, and thighs during normal walking, and FIG. 8 illustrates the rules for each position in the gait pattern.

Referring to FIG. 7, in one gait cycle, a stance phase is a section where a foot is in contact with the ground, and a swing phase is a section where the foot is lifted off the ground.

Within each gait cycle, there are three tasks as below.

1. Weight Acceptance

This period has an initial contact section (the foot first touching the ground) and a loading response section (a moment when the sole of the foot hits the ground).

2. Single Limb Support

This period includes a midstance section when the sole of the foot touches the ground and the foot of the opposite leg leaves the ground and a section when the heel is lifted and the opposite leg swings.

3. Limb Advancement

This period is when the other foot is planted, and includes the free swing, in which the toes of the front foot lift off the ground as the back foot leaves the ground, the mid swing, in which the foot that was in contact with the ground first begins to lift off the ground while the feet remain together, and the terminal swing, in which the heel of the front foot begins to touch the ground as the back foot pushes off the ground.

The gait pattern as described above is a normal gait pattern, and patients are trained to obtain this normal gait pattern.

However, in the case of patients who have difficulty walking normally, the patient's feet may become out of synchronization with each cycle during gait training. For example, in the mid-stance cycle, when the heel of the back foot is supposed to drop, the heel may not drop but remain on the floor. This is because the patient's body does not follow a normal gait pattern, and the robot of the present disclosure may forcibly bend the knee joint or ankle joint to match the patient's gait pattern at this time, causing the heel of the hind foot to come off the floor. All of these forced joints may be accomplished in a cycle, allowing patients during gait training to be forced to have a semblance of a normal gait pattern despite their physical limitations.

Below, a system control unit that effectively, accurately, and efficiently performs gait training for rehabilitation training of the patient as described above is described.

The system control unit in the present disclosure controls the gait training apparatus largely through software and partially through hardware.

These system control unit operates based on hardware called computer systems for executing software. Therefore, the system control unit according to the present disclosure may have all or part of a general computer system. The software uses a plurality of AI models to predict a patient's gait evaluation index from initial gait status information or gait training result information after gait training, establish an individual training plan for the patient, based on the gait evaluation index, evaluate the training result based on training result data according to the training result, and report the training result based on the evaluation of the training result.

According to the present disclosure, training of a general deep learning-based model is performed on a model for each function, and training using the trained models and applying the same are performed to establish, execute, evaluate and report a training plan for a patient, and in this process, an update of the training plan for long-term training is performed.

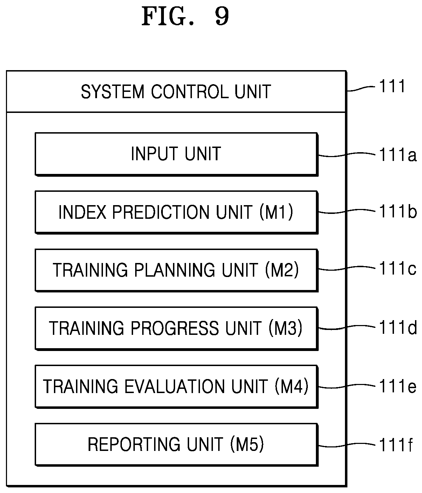

FIG. 9 illustrates functional parts based on the software and hardware structure of the system control unit 111 according to the present disclosure.

As illustrated in FIG. 9, the system control unit 111 may include an input unit 111a, an index prediction unit 111b, a training planning unit 111c, a training progress unit 111d, a training evaluation unit 111e, a reporting unit 111f, etc.

The input unit 111a may include at least a keyboard, or an input unit 111a including a keyboard, a webcam, and a communication unit, and an index prediction model M1 that predicts a patient's gait evaluation index required for establishing a patient's training plan.

The training planning unit 111b may include a training planning model M2 that plans training for individual patients according to patient symptoms by referring to indices predicted by the index prediction unit 111b.

The training progress unit 111d may include a training progress model M3 that monitors the training status according to the training plan and generates feedback on the training status to guide the patient and medical staff.

The training evaluation unit 111e may include an evaluation model M4 that evaluates the results of training performed by the training progress unit 111d and predicts future trends.

The reporting unit 111f may include a reporting model M5 that reports (reports) the results of training obtained from the training evaluation unit 111e to patients and training personnel in various forms.

In addition to the elements listed above, the system control unit 111 may further include other additional software and/or hardware depending on the design of the system control unit 111.

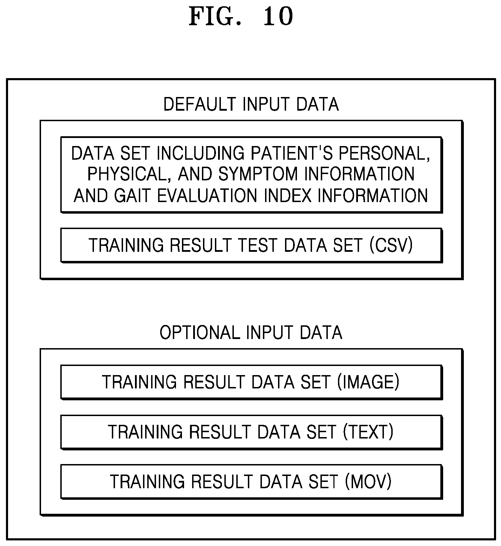

FIG. 10 is a schematic flowchart of a training process by the system control unit 111 in an AI-based gait training provision method according to the present disclosure, the method being performed by the exercise system 100 including the system control unit 111 described above.

Referring to FIG. 10, in the case of a new patient during the training process, after going through a data preparation operation by a data input process (S11) and an initial gait evaluation index generation process (S12), the process proceeds to the next operation S13. In the case of an already registered patient, the process proceeds to operation S13 after verifying personal information and calling up the existing gait evaluation index stored in the system.

In operation S13, a training plan is generated using initial or existing gait evaluation metrics.

In operation S14, the patient's gait training is performed according to the training plan described above.

In operation S15, an intermediate gait evaluation index is generated based on the current gait training results.

In operation S16, the training results are analyzed by comparing the current gait evaluation index based on the current training results with the existing gait evaluation index.

Operation S17 is an operation of forming and presenting various forms of reports based on the analyzed training results. After completing this operation, it is determined whether to continue training and training is either continued or ended. When it is selected to continue training, the process may proceed to operation S13 or S14 depending on whether the training plan has been updated.

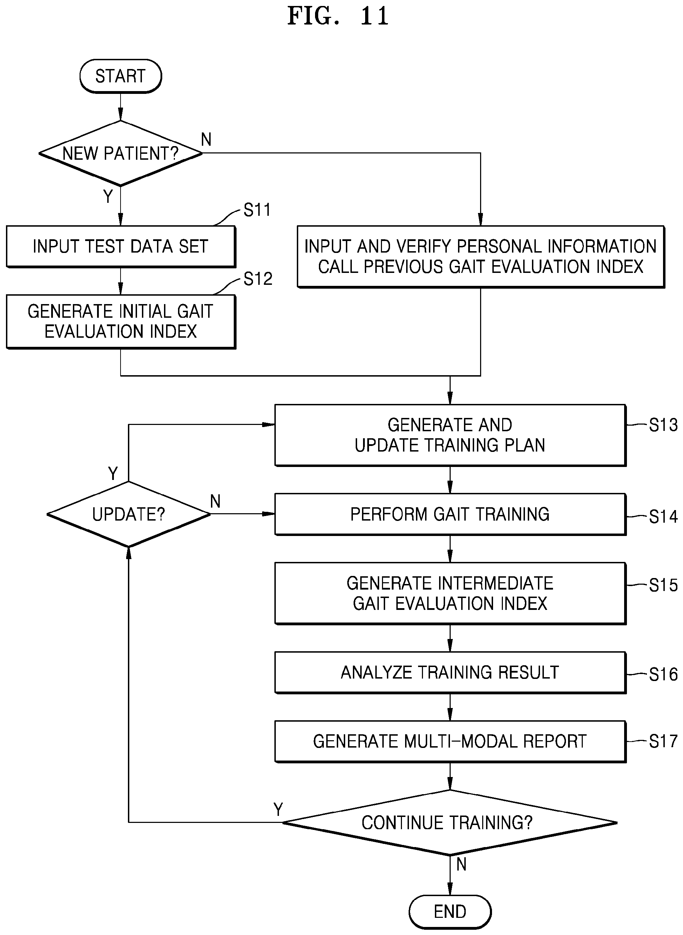

FIG. 11 is a flowchart of a more specific training process for new training patients.

Each operation schematically described in FIG. 11 may use, for example, five types of models M1, M2, M3, M4, and M5, in a step-wise manner, to perform gait training on the patient once or a number of repeated times. According to another embodiment, some of the models may be excluded.

When training begins, new patients begin with the process of operation S11 below, while patients who are already registered continue with the process from the operations of creating and updating a training plan in operation S13 below, after verifying and selecting their own information. That is, as described above with reference to FIG. 7, operations S11 and S12 are processes that are performed once initially, and thereafter, when training begins, a patient whose personal information is specified performs the training process from operation S13.

Operation S11: An initial test data set for gait training for one patient is input.

S12: The input initial test data set is input into the gait evaluation index prediction model M1 to generate an initial gait evaluation index.

S13: The gait evaluation index is input into the training plan generation model M2 to generate a training plan. In this case, when this process is not an initial training plan generation process but a generation process during an iterative training process, the training plan from the preceding process is updated.

S14: Once a training plan is generated or established, the patient's gait training is performed using the plan. This training is conducted by a gait training apparatus with the patient on board. During the training process, monitoring of the training status is performed, and information is provided to the patient according to the training status, and encouraging audio information and/or text information is presented. In the monitoring process, a real-time video of the patient's gait is acquired through a webcam or a Kinect sensor, which is included as components of the training-status detection unit, and from this, a machine learning-based model is used to extract the patient's major joint locations, such as key points, etc. to display the skeleton, and extract joint angle information and walking trajectories.

This information may be presented via the status display monitor 113 (FIG. 4) or alternatively, may be presented to the patient via the HMD 115 (FIG. 4) worn by the patient. The model M3 applied here may be a conversational model, such as a chatbot, for example.

S15: In this operation, an intermediate gait evaluation index is created based on the current gait training results. In this operation, the gait evaluation index prediction model M1 used in the initial gait evaluation index prediction above is applied.

S16: In this operation, the training results are analyzed by comparing the current gait evaluation index based on the current training results with the existing gait evaluation index. Here, the trained training analysis model M4 is used to analyze the training results.

S17: This operation is to create and present various forms of reports based on the analysis of the training results. In this operation, a model trained to generate reports from the training results, for example, a multimodal model M5, may be applied.

After this operation, it is determined whether to continue training, and when continuing training is selected, it proceeds to operation S13 or S14. When the current training result requires an update to the training plan, it proceeds to S13, and otherwise, when training is performed again with the current training plan, S14 proceeds directly to the gait training process.

Hereinafter, the AI models M1, M2, M3, M4, M5, etc. which are included in each portion of the system control unit, are described.

I. Model 1 (M1)

Model 1 (M1) is a model that predicts the patient's gait evaluation index and may be either a recurrent neural network (RNN) model or a long short-term memory model (LSTM).

One embodiment according to the present disclosure applies LSTM. The LSTM is a type of RNN and is a model designed to solve long-term dependency problems, and has a structure that may effectively learn sequence information of data.

When training the LSTM, the dropout and early stopping techniques are applied. Dropout is one of the regularization techniques for preventing overfitting of a neural network. It randomly disables (drops) some neurons during the learning process to prevent the model from overfitting to a specific pattern, and this technique may be applied to the embodiment of the present disclosure. Early stopping is a technique to prevent overfitting while a model is learning, which stops learning early when the performance on the validation data does not improve.

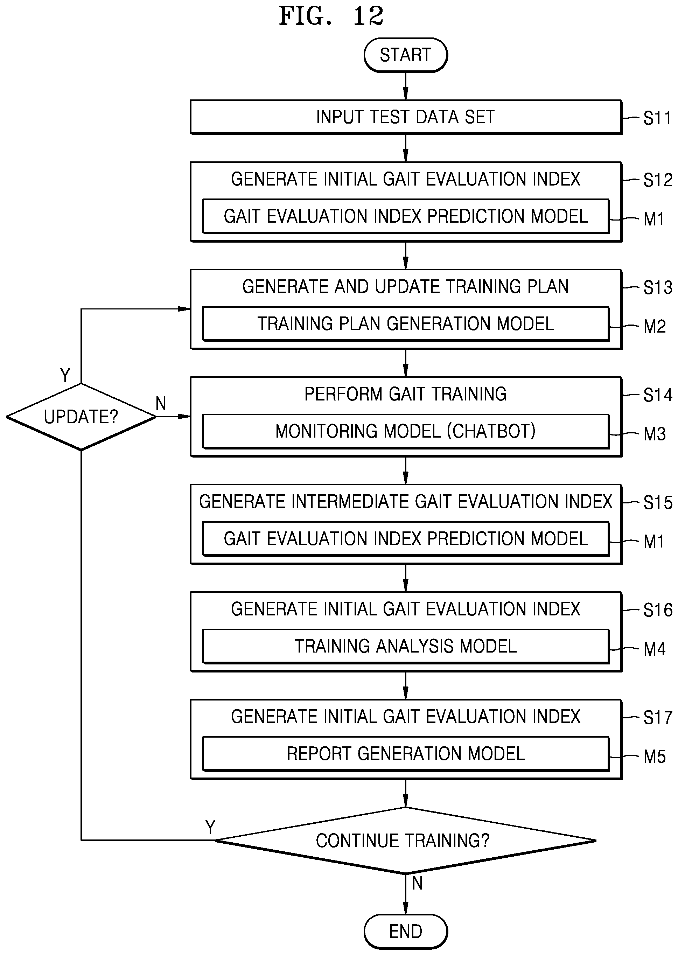

FIG. 12 illustrates the composition of a data set from a patient for generating an initial gait evaluation index through the gait evaluation index prediction model M1.

Default input data is default information entered into the exercise device 100 in the treatment hospital, and this must be managed in compliance with personal information protection procedures.

This data set includes patient information data, such as patient's personal information, patient's symptom information, gait evaluation indexes determined by medical staff (functional ambulation categories (FAC)), and the berg balance scale.

The patient's personal information includes age, sex, height, weight, leg length, foot size, and symptoms, and the symptoms information includes patient information such as symptoms and references for each symptom, date of symptom onset, and lower extremity symptoms (both feet, left foot, right foot).

In addition, it includes clinical evaluation indices using diagnosis results of medical staff, for example, clinical evaluation tools such as FAC, etc. based on medical staff diagnosis, and data from prior gait training.

Also, gait information data includes, as a data set measuring the results of gait training while walking on flat ground before boarding a gait rehabilitation training robot, information on upper body inclination and lower body joint angle during a gait. Also, as a data set measuring the results of gait training after boarding the gait rehabilitation training robot, an exercise system onboard training result data set according to the present disclosure is included. This includes average ground reaction force (GRF), center of pressure (COP), body weight support (BWS), lower extremity joint angle, and left-right balance information for each gait cycle (0 to 99%) during walking.

Also, as optional input data, information provided by the hospital or patient, such as doctor comments, gait images, gait training videos (MOVs), and training measurement information, for example, GRF information measured during walking on a GRF measurement pad, COP-based average (butterfly diagram) image information during walking, and upper body tilt information, are included.

Therefore, Model 1 (M1) is trained to input the data described above and output gait evaluation indices.

FAC, one of the input data, has six indicators and may be organized as follows.

FAC 0: Unable to walk - Incapable of walking.

FAC 1: The ability to walk is present, but walking ability is very limited and walking assistance is required.

FAC 2: The ability to walk is weak and walking assistance is required.

FAC 3: Partial recovery of walking ability, but requires some assistance with walking.

FAC 4: Walking ability is almost restored, and independent walking is possible without assistance.

FAC 5: Functional walking ability is fully restored and the patient can walk independently without assistance.

Meanwhile, the BBS consists of several items to evaluate the ability to maintain balance while performing various activities. These items include the ability to stand, transition from sitting to standing, maintain sitting to standing, and turn the head while standing, and each item is rated on a scale from 0 to 4, for a total score of 56, with higher scores indicating better balance.

Model 1 predicts the gait evaluation index of patients undergoing gait rehabilitation training by applying the grade reflecting the gait evaluation index standards (FAC, BBS, etc.) applied in the medical field, and this may be an RNN model or an LSTM model.

As a model training method to increase the judgment resolution with respect to the FAC (applying 6 levels), the time-series gait training result data (3 minutes of training for each of flat ground and stair walking) obtained from a non-disabled group (multiple people) using the exercise system of the present disclosure is set as the supervised learning model group (correct answer: 100%), and the time-series gait training result data with a walking disabled person on board are digitized from 0 to 100% using an anomaly detection model based on time series information, so that the walking grade may be determined.

The model's output data may vary depending on the design. However, when the FAC grade is applied, the measurement value (%) reflecting the anomaly detection model based on the time series information with respect to the measurement value of the disabled corresponding to the non-disabled measurement value (100%) may be compared and determined as below.

For example,

$$0 - 20\% = FAC\ 0$$

$$21 - 40\% = FAC\ 1$$

$$41 - 60\% = FAC\ 2$$

$$61 - 70\% = FAC\ 3$$

$$71 - 80\% = FAC\ 4$$

$$81 - 100\% = FAC\ 5$$

In cases where the FAC grade is not applied, the measurement value (%) reflecting the anomaly detection model based on the time series information with respect to the measurement value of the disabled corresponding to the non-disabled measurement value (100%) may be directly applied and relatively more specifically determined.

An example of the model performed as above is described below.

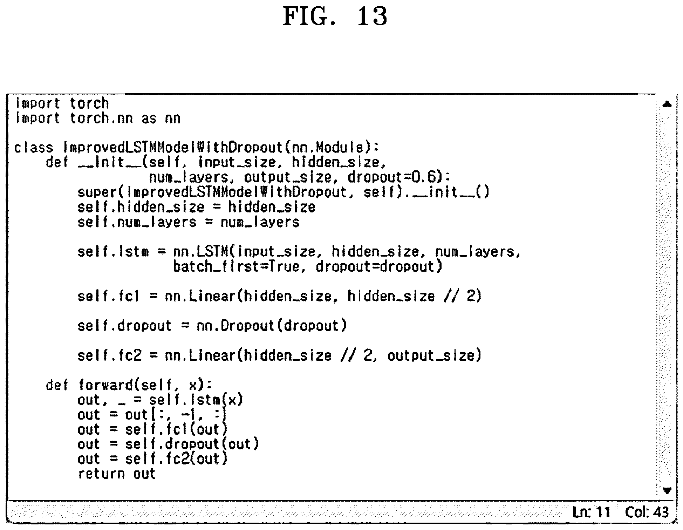

FIG. 13 illustrates a code snippet for the initialization of the ImprovedLSTMModelWithDropout class, which improves the LSTM model by adding a dropout layer. This code was written using PyTorch.

The ImprovedLSTMModelWithDropout class defines the LSTM model by inheriting nn.Module.

This class has initialization methods "__init__" and "forward" and the parameters of the initialization methods are as follows.

- input_size: Number of features in input data
- hidden_size: Size of the LSTM hidden state
- num_layers: Number of LSTM layers
- output_size: Number of features in the output data
- dropout: Dropout rate

Also, its instance variables are as follows.

- self.lstm: Defining the LSTM layer
- self.fc1: Defining the first fully connected layer
- self.dropout: Defining the dropout layer, and
- self.fc2: Defining the second fully connected layer.

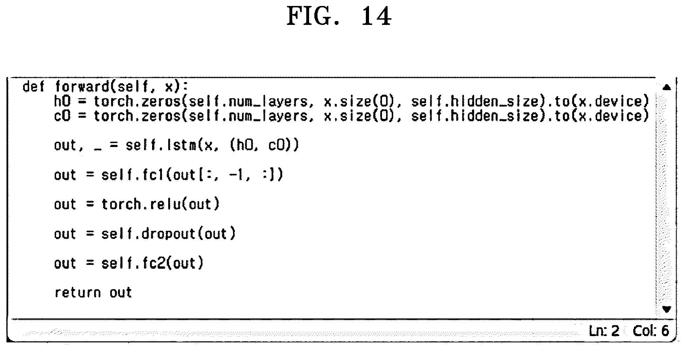

FIG. 14 illustrates a code snippet for defining the forward propagation process of the model.

This involves a series of operations that process the input data to generate final prediction values, with each operation transforming the input data to incrementally generate model prediction values.

Also, the main processing operations of the forward method are as follows.

(1) Forward Operation 1:

**h0 = torch.zeros(self.num_layers, x.size(0), self.hidden_size).to(x.device)**
**c0 = torch.zeros(self.num_layers, x.size(0), self.hidden_size).to(x.device)**

- In this operation, the initial hidden state (h0) and cell state (c0) are defined. Objects h0 and c0 are tensors representing the initial hidden state and cell state of the LSTM network, respectively. These are state values set before the network processes the first sequence and provide the initial state for each layer and element of the LSTM. Also, the torch.zeros function is used to create a tensor with all elements initialized to 0. These objects, i.e. the tensors, have a specific batch size (batch size, x.size(0)) and the size of the hidden state (self.hidden_size), and their dimensions are set according to the number of network layers (self.num_layers). Next, the generated tensor is moved to the same device (e.g., a central processing unit (CPU) or a graphics processing unit (GPU)) as the input data x by using the to (x.device) method.

(2) Forward Operation 2:
**out = self.lstm(x, (h0, c0))**
In this operation, the input data x and the initial state (h0, c0) are passed to the LSTM layer for processing. Here, x is the input data tensor (batch size, sequence length, and number of input features), out: the output of the LSTM layer. It is a tensor of the form (batch size, sequence length, hidden state size).
(3) Forward Operation 3:
**out = self.fc1(out[:, -1, :])**
out[:, -1, :]: Using only the output of the last time step of LSTM. This output passes through the first fully connected layer (self.fc1).
(4) Forward Operation 4:
**out = torch.relu(out)**
The ReLU activation function is applied to the output of the first fully connected layer. This improves the expressive power of the model by adding nonlinearity.
(5) Forward Operation 5:
self.dropout(out)
Some neurons are randomly disabled by applying dropout.
(6) Forward Operation 6:
**self.fc2(out)**
Output of the dropout layer is passed to the second fully-connected layer (self.fc2) to generate the final prediction value.
(7) Forward Operation 7:
**return out**

In this operation, the final prediction value of the model is returned.

In this way, the forward method defines the process by which input data is transformed into the final output through the LSTM, the fully connected layer, the activation function, and the dropout, with each operation gradually transforming the data to generate the final prediction.

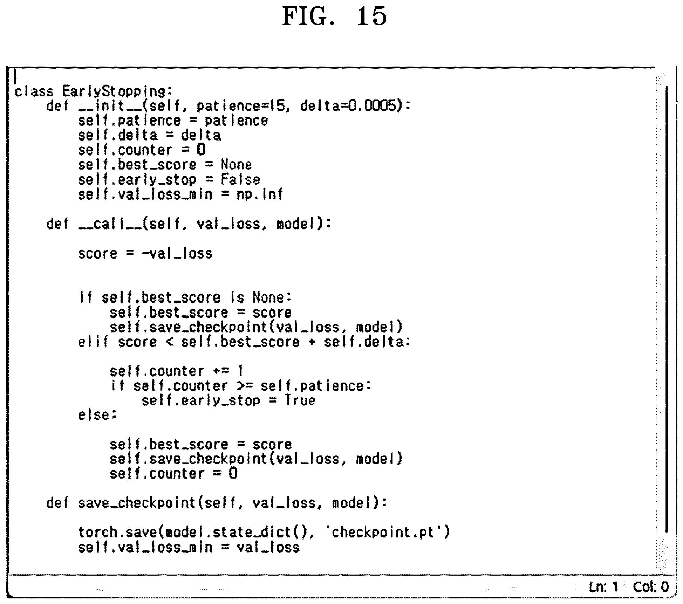

FIG. 15 illustrates a code snippet for the definition of an early stopping class.

This class is used to prevent overfitting and support efficient learning during the training process of machine learning models, and its main components and functions are as follows.

(1) Initialization method (_init_)
def_init_(self, patience=15, delta=0.0005):

- **patience (int):** An instance variable that sets how many epochs to wait for when performance does not improve. The default value is 15, which is passed as a parameter.
- **delta (float):** An instance variable that stores the minimum change to be recognized as a performance improvement. This value defines how much the validation loss must improve compared to the previous minimum loss. The default value is 0.0005, which is also passed as a parameter.
- **best_score (float):** An instance variable that stores the best observed performance score (negative value of validation loss).
- **early_stop (bool):** An instance variable that stores a flag indicating whether to terminate early.

- **val_loss_min (float):** An instance variable that stores the minimum observed validation loss.
- **counter (int):** An instance variable that stores the number of epochs at which performance does not improve.

(2) Call method (__call_)

```
def _call_(self, val_loss, model):
```

- **val_loss (float):** Validation loss at the current epoch.
- model (torch.nn.Module): The PyTorch model object being evaluated.

This method checks for early stopping conditions by evaluating the model's validation loss after each epoch. When the performance does not improve by more than delta from the previous best, a counter is incremented, and when this counter reaches patience, the early_stop flag is set to True to stop training. However, when the performance improves, the counter is reset and the model's state is saved as a checkpoint.

(3) Checkpoint save method (save_checkpoint) **def** save_checkpoint(self, val_loss, model):

This method saves the state of the model to a file (checkpoint.pt). This is used when the model is reloaded later or when it is desired to keep a state with better performance and updates val_loss_min with the latest validation loss value.

The main purpose of this early stopping class is to help with efficient learning, prevent overfitting, reduce unnecessary training time, and maintain the generalization ability of the model. This early stopping is an important technique in many machine learning scenarios, especially useful in deep learning models that require high computational costs.

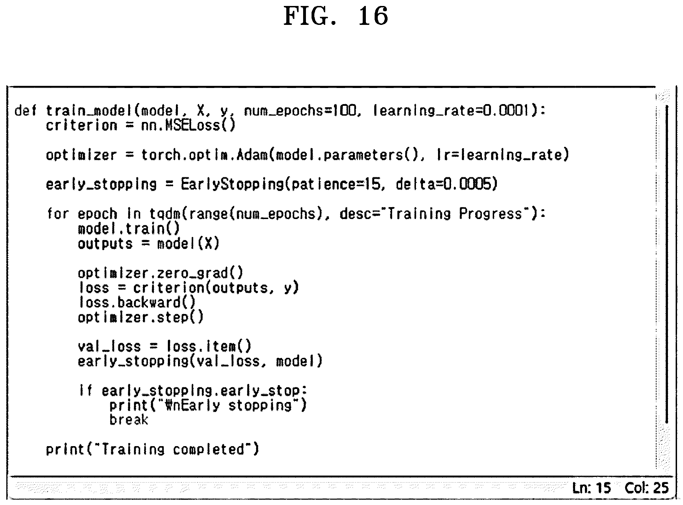

FIG. 16 illustrates a code snippet for the definition of train_model, a function for model training.

def train_model(model, X, y, num_epochs=100, learning_rate=0.0001):

- The function train_model is used to train a model, and takes model (PyTorch model object to train), X (input data tensor), y (output data tensor), num_epochs (number of epochs to train, default 100), and learning_rate (learning rate, default 0.0001) as parameters.

(1) Loss function: Mean square error (MSE)

```
criterion = nn.MSELoss()
```

- A loss function is defined. Here, the MSE loss function is used. MSE is the average of the squares of the differences between the prediction value and the actual value, and is mainly used in regression problems.

(2) Creating an optimizer class

```
optimizer = torch.optim.Adam(model.parameters(), lr=learning_rate)
```

- An Adam optimizer object to update the model's weights is created by using the torch.optim.Adam class. The optimizer adjusts the model's weights along the gradient by using the learning rate learning_rate.

(3) Creating an early stopping class instance

```
early_stopping = EarlyStopping(patience=15, delta=0.0005)
```

- An instance of the EarlyStopping class for early termination is created. Patience is the number of epochs to wait for without any improvement in loss, and delta is the minimum amount of change to be considered an improvement.

(4) Training loop

```
for epoch in tqdm(range(num_epochs), desc="Training Progress"):
```

- This is a loop in which training progresses. It repeats for a total of num_epochs and uses the tqdm library to visually display the training progress.

(4.1) Setting the model to training mode

```
model.train()
```

- The model is switched to a training mode. This is necessary to make layers like dropout or batch normalization operate differently in the training mode.

(4.2) Generating prediction values
outputs = model(X)

- Input data X is passed to the model to generate prediction values. Outputs are the model's prediction results.

(4.3) Initializing the gradient of the optimizer
**optimizer.zero_grad()**

- The gradient buffer of the optimizer is initialized, which is needed to initialize the gradient before computing the gradient via backpropagation.

(4.4) Loss calculation
**loss = criterion(outputs, y)**

- The loss between the prediction values (outputs) and the actual values (y) is calculated. Here, the loss value is calculated using the MSE loss function.

(4.5) Calculating the loss gradient
loss.backward()

- The gradient of the loss is calculated through backpropagation, and the gradient of each parameter is calculated in each operation.

(4.6) Weight update
optimizer.step()

- The model's weights are updated, based on the calculated gradient, by using the optimizer.

(4.7) Saving current loss value
val_loss = loss.item()

- The current loss value is converted to a scalar value and is saved. This will subsequently be used as a criterion for early termination.

(4.8) Checking early stopping conditions
early_stopping(val_loss, model)

- The early stopping condition is checked by using the loss value of the current epoch. The early_stopping object tracks the number of epochs without improvement and stops training the model when the condition is met.

(4.9) Outputting early stopping message
**if early_stopping.early_stop: print("\nEarly stopping")**

- In this operation, when early_stopping.early_stop is True, an early stopping message is output and the loop is exited, stopping training.

(5) Outputting training completion message
**print ("Model training completion")**

- Finally, a message indicating that training is complete is output.

The code snippet explained above in a step-wise manner implements a basic framework for training a PyTorch model through early stopping. The use of the MSE loss function and the Adam optimizer is common in regression tasks, and the early stopping mechanism helps prevent overfitting by stopping training when the validation loss no longer improves significantly.

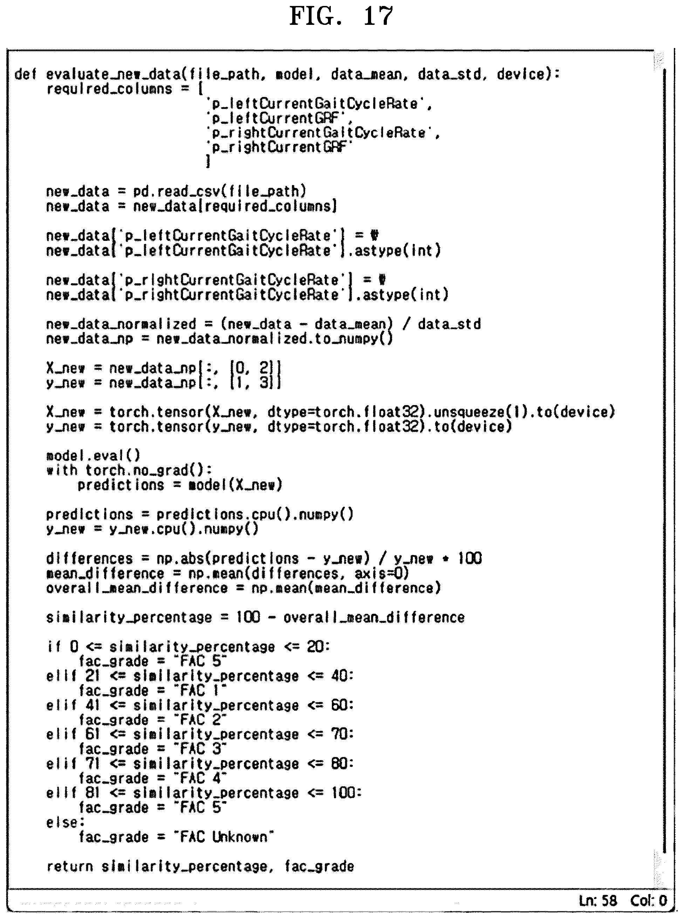

FIG. 17 illustrates a code snippet for a data evaluation function that evaluates new data from a gait training patient and predicts the FAC for that patient.

This example function uses a PyTorch model to generate a prediction value for the new data and compares the prediction value with the actual value to calculate the similarity.

(1) Data loading:
**def evaluate_new_data(file_path, model, data_mean, data_std, device):**
The function evaluate_new_data reads a CSV file from file_path, selects only the necessary columns, and converts it to a DataFrame. The necessary columns are, for example, the left current gait cycle rate (p_leftCurrentGaitCycleRate), the left current GRF (p_leftCurrentGRF), the right current gait cycle rate (p_rightCurrentGaitCycleRate), and the right current GRF (p_rightCurrentGRF).
# Data preprocessing:
**new_data['p_leftCurrentGaitCycleRate'] =**
**new_data['p_leftCurrentGaitCycleRate'].astype(int)**
**new_data['p_rightCurrentGaitCycleRate'] =**
**new_data['p_rightCurrentGaitCycleRate'].astype(int)**
p_leftCurrentGaitCycleRate and p_rightCurrentGaitCycleRate are converted to integers and then normalization is performed.
(2) Preparing model input data:
The input data (X_new) and the output data (y_new) are separated from the normalized data. The input data includes p_leftCurrentGaitCycleRate and p_rightCurrentGaitCycleRate, and the output data includes p_leftCurrentGRF and p_rightCurrentGRF.
(2) **Model prediction:**
Input data is converted into PyTorch tensors and passed to the model to generate the prediction value. The model is set to an evaluation mode and gradient computation is disabled using the method torch.no_grad().
(3) Result calculation:
The difference between the prediction value and the actual value is calculated in percentage units to obtain the average error rate. The similarity is calculated based on this.
(4) FAC grade determination:
The patient's FAC grade is determined based on the similarity. For example, when the similarity is 0-40%, it follows the rule of FAC 1, etc.

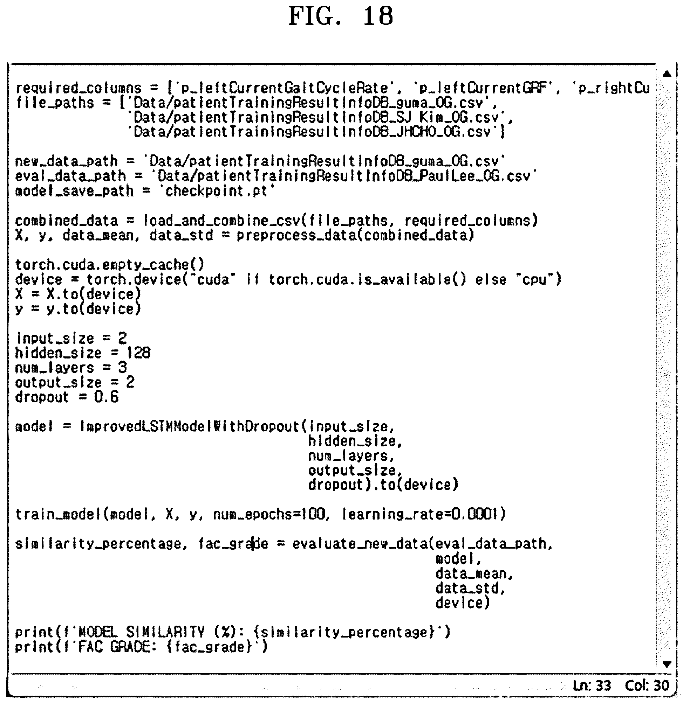

FIG. 18 illustrates a code snippet with respect to execution of the LSTM model described above.

(1) Specifying required columns and setting file path
In this process, the required data columns (required_columns) and file path (file_path) are first specified.

- file_paths: Paths to multiple CSV files to be used for model training.
- new_data_path and eval_data_path: Paths for new data and evaluation data.
- model_save_path: Specifying the checkpoint file path for the trained model.

(2) Data loading and preprocessing
**combined_data = load_and_combine_csv(file_paths, required_columns)**
**X, y, data_mean, data_std = preprocess_data(combined_data)**

- load_and_combine_csv: Function to read and combine data from multiple CSV files.
- preprocess_data: Preprocessing the combined data and returning the input data X, output data y, data mean data_mean, and data standard deviation data_std.

(3) Processor (GPU or CPU) usage setting
**device = torch.device("cuda" if torch.cuda.is_available() else "cpu")**

- In this process, the GPU memory is released, - when the GPU is available, CUDA is set as the device, otherwise CPU is set as the device, and then the data is transferred to the set device.

(4) Model initialization and training
An improved LSTM model with dropout is created using the ImprovedLSTMModelWithDropout class and the model is trained using training data by using the train_model function. Here, according to the present embodiment, the training is performed for 100 epochs, and the training rate is set to 0.0001.
(5) Data Evaluation
In this operation, a data evaluation function (e.g., the evaluate_new_data function described above) evaluates the data, and the results are stored in the similarity_percentage and FAC grade (fac_grade) variables.

In this operation, data evaluation is performed by a data evaluation function, for example, the function evaluate_new_data described above, and the results are stored in the similarity_percentage and fac_grade variables.

This code snippet loads and preprocesses data from the CSV file containing information used for FAC assessment of a given patient's gait training, trains the LSTM model, evaluates new data using the trained model, and calculates the similarity between the predicted result and the actual result to determine the FAC grade. This may be used as initial data for gait training of patients, such as gait analysis, or to evaluate advanced FAC of patients undergoing gait training thereafter.

II. Model 2 (M2)

Model 2 is an AI agent that automatically generates a patient symptom-specific gait training plan for a gait rehabilitation training robot in an AI-based robot-assisted orthopedic exercise system according to the present disclosure, and this model may be, for example, a Transformer model, and according to another embodiment, LSTM or a gated recurrent unit (GRU) may also be applied.

Input data for this Model 2 includes 1) patient information, 2) prior gait training result data, and 3) gait training standard protocol and training plan definition data.

The patient information includes gender, weight, foot size, year of birth, and FAC (gait evaluation index) grade assigned to the patient, which are input when the patient is registered in the training system. The prior training result data includes a data set of training protocols such as stair climbing/descending, slope climbing/descending, etc., training time, and training parameters such as stride length, stride height, initial contact angle, toe off angle, ground reaction force, gait cycle, etc. Also, the gait training standard protocol and training plan definition data are data determined by medical staff.

Model 2 receives data, such as the FAC of the patient currently diagnosed, patient information, and current training result information, and derives (outputs) a training plan, such as a training protocol for walking on flat ground, climbing/descending stairs, climbing/descending slopes, etc., training time, training parameters, etc., and Model 2 may be a multi-input, multi or single output model.

According to another embodiment, the inputs may be current gait evaluation indices and patient information, and the output may be a standard training model group (classification), and according to yet another embodiment, the inputs may be current gait evaluation indices, patient information, and training information, and the output may be patient-tailored training plan information.

When applying the AI autonomous judgment training plan, the output data of Model 2 is a training plan definition data set that reflects result information of previously performed training for each patient. In real-time training plan modification reflecting training status information during training for each patient, settings of training time, training parameters, changes in training protocols, and stopping/starting of the robot are included.

The M2 model for generating an AI autonomous judgment training plan is an AI agent that predicts training parameters by using the patient's gait training data and may be generated and trained by Python and PyTorch based on it, like the model M1 described above. For this purpose, a number of libraries, including PyTorch, may be imported as follows.

- import os: Module for file and directory operations
- import torch: Library for basic tensor operations and neural network establishment.
- import torch.nn as nn: Neural network module (e.g. layer definition)
- import torch.optim as optim: Optimization algorithm for model training (e.g. Adam, SGD, etc.)
- from torch.utils.data import DataLoader, TensorData set: Modules for data processing (batch generation, etc.)
- import pandas as pd: Library for creating and manipulating data frames

The Python code that imports the libraries described above sequentially includes the following processes for declaring and training the M2 model.

(1) Data input and data frame generation

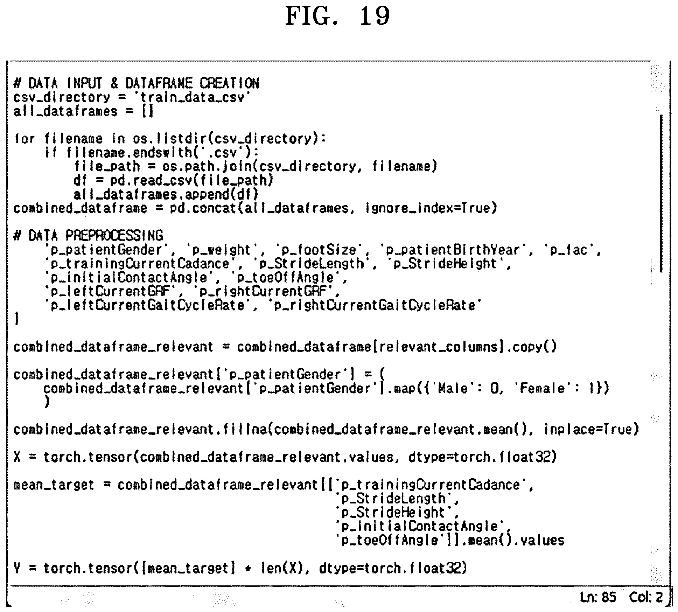

FIG. 19 is a code snippet with respect to generation of data inputs for training the model and a data frame in which the data inputs are accumulated, from among the entire Python codes for generating the M2 model.

The M2 model is an instance of a Transformer model that utilizes the PyTorch's neural network module, nn, to predict training parameters using the patient's gait training data. As described sequentially below, input data is provided in the form of multiple CSV files, and the input data are integrated into a two-dimensional data frame through a preprocessing process and then used as training data.

(1.1) Setting the directory path containing CSV files
csv_directory = 'train_data_csv'
(1.2) List Initialization
all_dataframes = []
(1.3) Reading all CSV files in directory and combining them into one data frame. for filename in os.listdir(csv_directory):

```
if filename.endswith('.csv'):
    file_path = os.path.join(csv_directory, filename)
    df = pd.read_csv(file_path)
    all_dataframes.append(df)
```

(1.4) Combining all data into one data frame
combined_dataframe = pd.concat(all_dataframes, ignore_index=True)
(2) Data preprocessing and tensor conversion
(2.1) Definition of columns

```
relevant_columns = [
  'p_patientGender', 'p_weight', 'p_footSize', 'p_patientBirthYear', 'p_fac',
  'p_trainingCurrentCadance', 'p_strideLength', 'p_strideHeight',
  'p_initialContactAngle', 'p_toeOffAngle',
  'p_leftCurrentGRF', 'p_rightCurrentGRF',
  'p_leftCurrentGaitCycleRate', 'p_rightCurrentGaitCycleRate'
]
```

The input data may include, as patient information, gender "p_patientGender," weight "p_weight," foot size "p_footSize," birth year "p_patientBirthYear," and FAC "p_fac," and as prior training result data, the number of walks per minute "p_trainingCurrentCadance," stride width "p_strideLength," stride height "p_strideHeight," initial contact angel "p_initialContactAngle," toe off angle "p_toeOffAngle," a current left foot GRF "p_leftCurrentGRF," a current right foot GRF "p_rightCurrentGRF," the number of left foot gait cycles "p_leftCurrentGaitCycleRate," and the number of right foot gait cycles "p-rightCurrentGaitCycleRate."

(2.2) Copying data frame to selected columns

combined_dataframe_relevant = combined_dataframe[relevant_columns].copy()

(2.3) Data preprocessing

combined_dataframe_relevant['p_patientGender'] = combined_dataframe_relevant['p_patientGender'].map({'Male': 0, 'Female': 1})
In the preprocessing process, a male "male" is converted to "0," a female "female" is converted to "1," and missing values (not a number (NaN)) are replaced with the mean value of each column by the method "fillna." combined_dataframe_relevant.fillna(combined_dataframe_relevant.mean(), inplace=True)

(2.4) Conversion to PyTorch tensor "X" and creation of tensor "Y"

mean_target = combined_dataframe_relevant[['p_trainingCurrentCadance', 'p_stepLength', 'p_stepHeight', 'p_initialContactAngle', 'p_toeOffAngle']].mean().values
The preprocessed data is converted to the PyTorch tensor "X," then the mean value, mean_target, of the target parameters to be predicted is calculated, and the tensor "Y" with the same target value for all samples is generated. Y = torch.tensor([mean_target] * len(X), dtype=torch.float32)

(3) Definition of Transformer-based model class

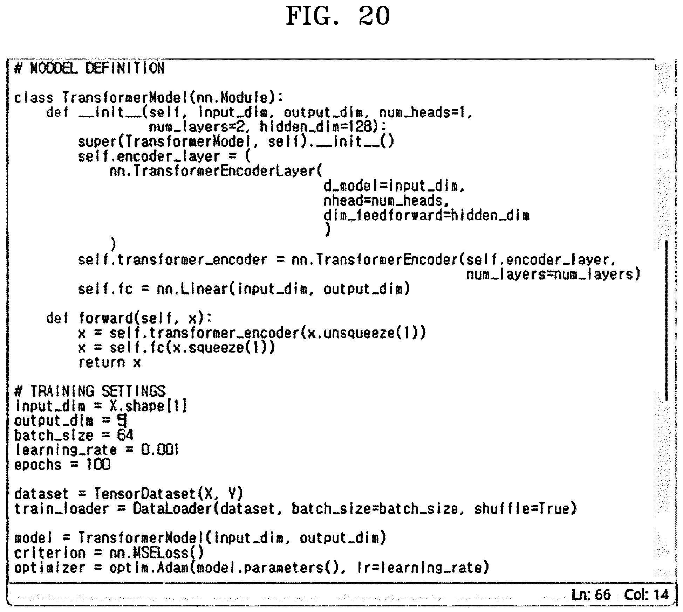

FIG. 20 is a code snippet with respect to declaration, training setting, model generation, etc. of the transformer model, from among the entire Python codes.
class TransformerModel(nn.Module):
A **TransformerModel** class is defined that inherits PyTorch's nn.Module.
The initialization method __init__ of **TransformerModel** is the constructor method of the class. It defines the structure of the model and each layer and calls the initialization method **super (TransformerModel, self).__init__** of the parent class to perform the initialization function of the base class. Its parameters are as follows.

- **input_dim:** Specifying the dimension (number of features) of the input data.
- **output_dim:** Specifying the output dimension (the number of parameters to be predicted) of the model.
- **num_heads:** Specifying the number of heads in multi-head attention. The default value is 1.
- **num_layers:** Specifying the number of Transformer encoder layers. The default value is 2.
- **hidden_dim:** Setting the dimension of the forward propagation network. The default value is 128.

**The class instance nn.TransformerEncoderLayer** defines self.encoder_layer , a Transformer encoder layer provided by PyTorch, as follows.
self.encoder_layer = nn.TransformerEncoderLayer(d_model=input_dim, nhead=num_heads, dim_feedforward=hidden_dim)
The parameters for this instance are as follows.

- d_model=input_dim: Input dimension.
- **head=num_heads:** The number of heads in multi-head attention.
- dim_feedforward=hidden_dim: Dimension of the forward propagation network.

The fully connected layer (fc) is defined by the method nn.Linear, where the parameter **input_dim** is the input dimension, and **output_dim** is the final output dimension and is the number of training plan parameters to predict.
Also, the forward propagation method forward of the **TransformerMode** preprocesses the input data and returns it. To do this, first, a dimension is added to the input data x, and then the input x with the added sequence dimension is passed to the Transformer_encoder to convert it into an encoded output. And after the sequence dimension is removed from the encoded output x by the method squeeze, the transformed data x is returned as the output through the fully connected layer.

(4) Training settings

As shown in FIG. 20, the hyperparameters in the settings for training are set as follows.

- input_dim = X.shape[1] # Dimension of input data (number of features)
- output_dim = 5 # Dimension of output data (number of parameters to predict)
- batch_size = 64 # batch size (number of data samples to learn at once)
- learning_rate = 0.001 # learning rate (size of change when updating weights)
- epochs = 100 # Number of learning repetitions

After this process, the data set and data loader are created as follows.

- data set = TensorData set(X, Y) # Define data set
- train_loader = DataLoader(data set, batch_size=batch_size, shuffle=True) # Create a data loader
- model = trasnformerMode(input_dim, output_dim): Creating an instance of the M2 model

In the following process, the model to be trained is initialized and the loss function and optimizer are set. According to the present embodiment, after the M2 model is initialized, as a transformer model, "MSE" is set as the loss function and "Adam" is set as the optimizer, and then, model training is performed.

(5) Model training

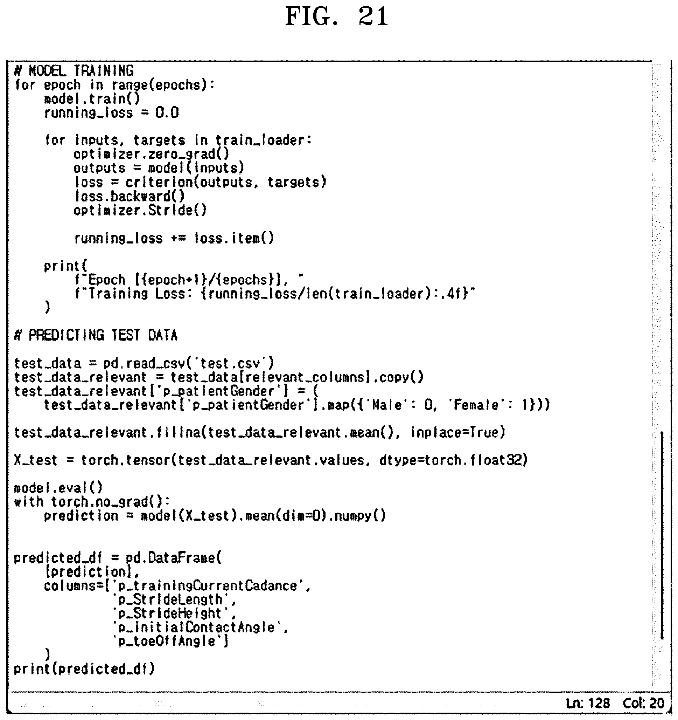

FIG. 21 is a code snippet for training the model and predicting test data.
As illustrated in FIG. 21, according to one embodiment of the present disclosure, the training of the model is repeatedly performed for 100 epochs pre-set in the operation above.

- **for epoch in range(epochs):** A loop to repeat the entire training process is started Epochs define the number of times to repeat training.
- **model.train():** The model is set to a training mode. This mode allows certain layers, such as dropout and batch normalization, to operate during training.
- **running_loss = 0.0:** A variable to store the total loss of the current epoch is initialized, and the average loss is calculated using this variable at the end of each epoch.

- **for inputs, targets in train_loader:** Input data (inputs) and target data (targets) are loaded in train_loader in units of batches. The data loader is responsible for repeatedly importing the data set in units of batches.
- **optimizer.zero_grad():** The gradient of the optimizer is initialized. Since PyTorch accumulates previous gradients by default, the gradients have to be initialized for each batch.
- **outputs = model(inputs):** The input data is passed to the model to calculate the prediction values (outputs), and the model's forward method is called in this operation.
- **loss = criterion(outputs, targets):** The loss between the prediction value and the actual target value is calculated. Here, criterion defines the loss function (e.g. MSELoss).
- **loss.backward():** Backpropagation is performed based on the loss to calculate the gradient for each parameter of the model.
- **optimizer.step():** The model's parameters are updated based on the gradient calculated using the optimizer.
- **running_loss += loss.item():** The loss values of the current batch are accumulated and added to running_loss, to calculate the average loss at the end of an epoch. Also,
- **print():** The average loss is printed for each epoch and running_loss is divided by the length of train_loader to calculate, format, and print the average. For example, when the real-time loss is 250.123 as the output of the 6th epoch out of a total of 100 epochs and the length of train_loader is 50, the output will be "Epoch [6/100], Training Loss: 5.0025."

The model training process is performed repeatedly, and the loss may be calculated and output for each epoch. Here, the optimizer and the loss function are used to update the model's parameters, and the progress of training may be monitored through the loss value.

(6) Predicting test data

As illustrated in FIG. 21, the prediction of test data using the M2 model obtained through the process above is also performed on the input data of the CSV file.

- test_data = pd.read_csv('test.csv') : Reading test data file
- test_data_relevant = test_data[relevant_columns].copy() : Selecting the necessary columns from the data in test data preprocessing

      test_data_relevant['p_patientGender'] = test_data_relevant['p_patientGender'].map({'Male': 0, 'Female': 1}): Converting gender data to numbers
      test_data_relevant.fillna(test_data_relevant.mean(), inplace=True) : Processing missing values
      X_test = torch.tensor(test_data_relevant.values, dtype=torch.float32): Converting test data to PyTorch tensor.

(7) Model prediction

      model.eval(): Setting the model to evaluation mode (disabling dropout, etc.)
      with torch.no_grad(): Disabling gradient computation
      prediction = model(X_test).mean(dim=0).numpy(): Calculating the prediction value and then calculating the mean.

(8) Outputting prediction results

      predicted_df = pd.DataFrame([prediction], columns=['p_trainingCurrentCadance', 'p_stepLength', 'p_stepHeight', 'p_initialContactAngle', 'p_toeOffAngle'])
      print(predicted_df) : Converting the prediction results to a data frame and outputting them

The model trained as above is used as a model to establish a patient-tailored training plan.

In actual usage, in order to establish a training plan by reflecting the information on the results of previously performed training, the most recent data is extracted from the existing training data, prediction is performed using the M2 model, and the result values after the prediction are input into new training to perform training.

When establishing the training plan using the M2 model, when there is patient's pre-training information, the patient's pre-training information is input into the M2 model to extract an autonomous training plan. And, if necessary, additional work is performed to improve reliability by inputting the information into the expert system.

The expert system includes a reference model trained according to theoretical criteria. This reference model has the same structure as the M2 model and outputs a data set defining the standard protocol and training plan for gait training determined by a medical professional as a reference training plan. This output matches the training plan establishment information corresponding to the patient information and determined gait grade evaluation indices. This reference model is a model trained using patient information (gender, weight, foot size, year of birth, FAC grade, etc.), training protocol, training time, and training parameters set by the medical staff.

This reference model may be applied as a model to determine the reliability of a pre-built AI autonomous judgment training plan establishment model and the M2 model described above. To this end, patient's pre-training information input into the pre-built AI autonomous judgment training plan establishment model and the M2 model described above is input into the reference model to extract a reference training plan.

This reference training plan is compared with the autonomous training plan output from the M2 model, and thus, the reliability may be determined, and according to the reliability, the autonomous training plan may be appropriately corrected. In actual training, either the reference training plan or the autonomous training plan may be selected. The reference model described above may be an optional element and may be applied as a means to more precisely compensate or correct the autonomous training plan.

According to another embodiment, the autonomous training plan may be corrected based on the reference training plan. This correction may also be performed by a final training plan model that outputs an optimal training plan from the outputs of the M2 model and the outputs of the reference model. The final training plan model may be trained using the data obtained by comparing the M2 model described above and the reference model.


III. Model 3 (M3)


Model M3 is a training progress model that monitors the training status and guides the training status during gait rehabilitation training. Model M3 may be, for example, a chatbot-type model. Input data for this model includes patient information, robot status information, and real-time input/output information during a gait. For this model M3, an AI agent based on one deep learning model selected from an MLP and an open vision-language model (VLM) may be applied.

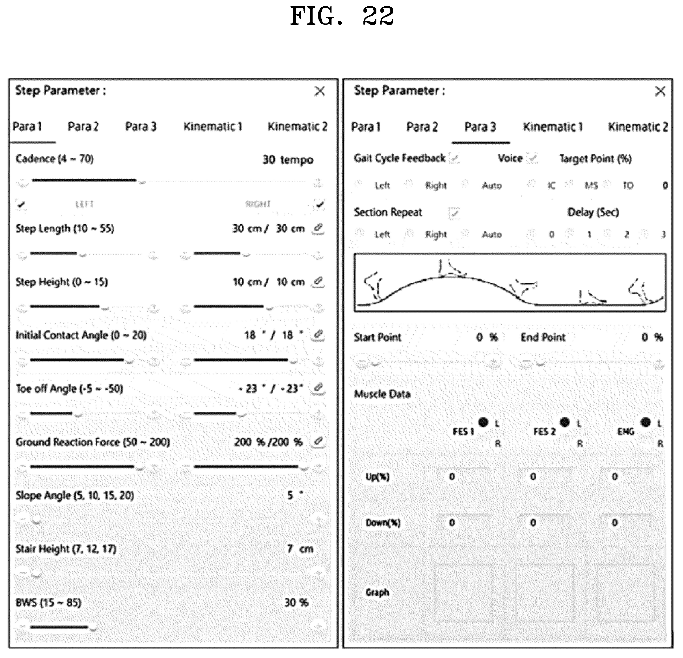

FIG. 22 illustrates a graphical interface showing various parameters of gait training.

Real-time input/output information during gait training includes training protocols, training parameters, and training data at regular step intervals detected in real time.

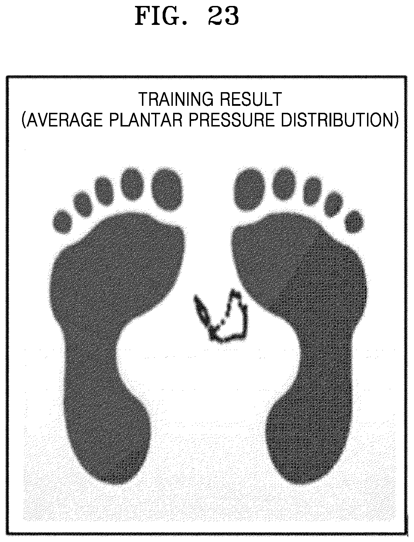

FIG. 23 is a butterfly diagram showing the average plantar pressure distribution of the patient during training as a result of the patient's gait training.

This training data may include data acquired in real time from a patient undergoing gait training. This data may include at least one piece of characteristic information from among GRF (%), BWS (%), upper body inclination or tilting (degree), joint angle (degree), cadence, left gait cycle (0-99%), right gait cycle (0-99%), stride length, ankle angle, oxygen saturation data, and heart rate data.

Training parameters may include step length (cm), step height (cm), initial contact angle (degree), and toe off angle (degree).

As a separate option, when a video recording device, such as a webcam, or a voice recording device with a microphone, is installed in the system, the patient's facial expression, voice and gesture information, and the therapist's voice and gesture information may be obtained and used as input data.

To apply these separate options, an image analysis unit may be provided that analyzes images from the camera to analyze the patient's facial expression or gesture, for example, an image analysis unit having an image analysis model trained to analyze the patient's facial expression or gesture from images.

The model M3 may be trained to receive data from the image analysis unit and generate output according to the patient's training status together with the input data.

The output data of such model M3 may include state information during training, i.e. feedback on real-time training data and corrections to current training parameters.

The status feedback may include audio feedback such as an AI voice, a beep sound, etc., status message output to a user interface, for example, a virtual reality HMD, indicated as sound or image or transmitted in text, such as "Hello, Mr. Smith" when starting training after a patient gets on board, "Your training is really good today" when training is in a similar pattern to the training data, "Press your left foot more" when the maximum GRF of the left foot is 10% or more lower than the maximum GRF of the right foot per 1 gait cycle, "Straighten your upper body " when the upper body is gradually leaning forward or to the side per 10 gait cycles, "Cheer up" when the average GRF value before 10 gait cycles is higher than the average value per minute of the current gait cycle, "Your heart rate is rising" when the heart rate increases sharply in 1 gait cycle, etc. Also, according to another embodiment, a real-time change of training parameters, such as increasing or decreasing the speed or stopping the robot, may be requested (required) through the user interface based on the monitoring result of the training status.

Training parameter modifications that may be included in the output data of the model M3 may include the step length, step height, initial contact angle, and toe-off angle as recommended training parameters capable of performing gait training close to the correct answer.

The model M3 may perform the function of a training support agent through real-time interaction with patients/therapists, and may provide training status information by having an AI agent compare and judge sequential training results and real-time training results during training.

The model M3 may apply the MLP, which is a type of feedforward neural network, as described below.

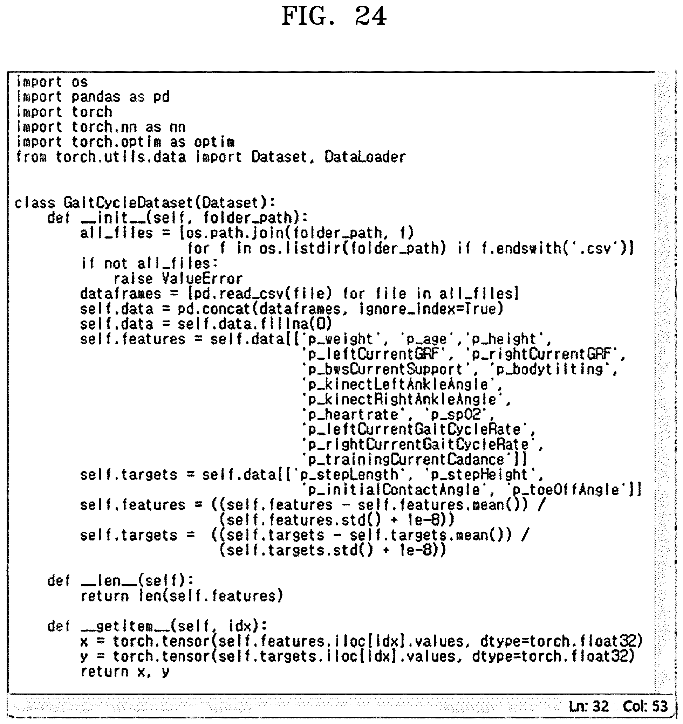

FIG. 24 is a code snippet of the declaration unit of the data set class for training the model M3.

The program for training the model M3 is written in Python, for example, and a number of libraries are imported as follows.

```
import os
import pandas as pd
import torch
import torch.nn as nn
import torch.optim as optim
from torch.utils.data import Data set, DataLoader
```

Referring to the descriptions above, the OS module for interacting with the operating system in Python is imported, and pandas, a Python library for data analysis and manipulation, is assigned as the alias "pd." Additionally, torch, which constitutes a deep learning framework as a basic module of the PyTorch library and supports tensor operations, automatic differentiation, GPU acceleration, etc., is also called. torch.nn, the neural network module of PyTorch, is imported and assigned as the alias "nn."

The GaitCycleData set class reads .csv files in a specified folder and consolidates the data into one. In this process, missing values are filled with 0, and feature data (input) and target data (output) are normalized and stored separately. This class provides a method __getitem__ which returns each data point as a tensor.

The data set described above has the following elements.

- Weight: p_weight
- Age: p_age
- Height: height'
- Left current GRF: p_leftCurrentGRF
- Right Current GRF: p_rightCurrentGRF
- Current BWS: p_bwsCurrentSupport
- Body tilt: p_bodytilting
- Left ankle angle: p_kinectLeftAnkleAngle
- Right ankle angle: p_kinectRightAnkleAngle
- Heart rate: p_heartrate
- Oxygen saturation: p_spO2
- Left current gait cycle: p_leftCurrentGaitCycleRate
- Right current gait cycle: p_rightCurrentGaitCycleRate
- Current training steps per minute: p_trainingCurrentCadance

The data set outputs step length (p_stepLength), step height (p_stepHeight), initial contact angle (p_initialContactAngle), and toe-off angle (p_toeOffAngle) as targets.

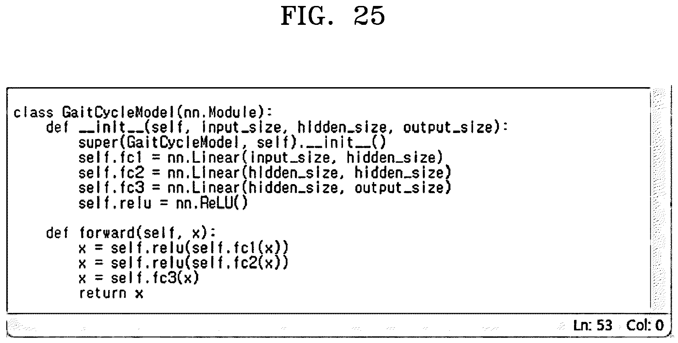

FIG. 25 is a code snippet defining GaitCycleModel, which is the Model M3.

Referring to FIG. 25, the GaitCycleModel class has an MLP structure. The Model 3 of the MLP structure is a feedforward neural network including an input layer, two hidden layers, and an output layer, and each hidden layer uses the ReLU activation function.

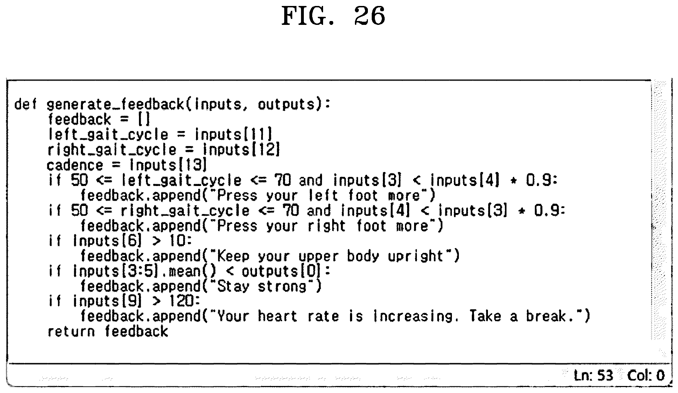

FIG. 26 is a code snippet of the feedback generation function, generate_feedback. The feedback generation function generates various feedbacks to be provided to pedestrians based on real-time input data and model output values.

For example, as described above, depending on the training status, the feedback may be provided to the patient, such as "Hello, Mr. Smith" when starting training after a patient gets on board, "Your training is really good today" when training is in a similar pattern to the training data, "Press your left foot more" when the maximum GRF of the left foot is 10% or more lower than the maximum GRF of the right foot per 1 gait cycle, "Straighten your upper body" when the upper body is gradually leaning forward or to the side per 10 gait cycles, "Cheer up" when the average GRF value before 10 gait cycles is higher than the average value per minute of the current gait cycle, "Your heart rate is rising" when the heart rate increases sharply in 1 gait cycle, etc.

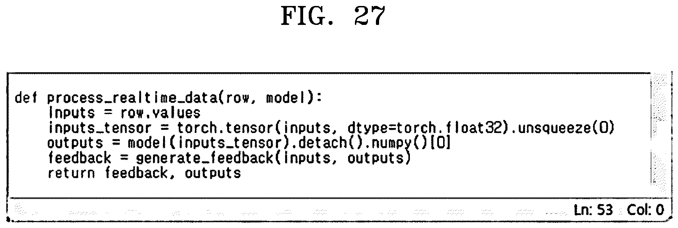

FIG. 27 is a code snippet for the real-time data processing function process_realtime_data.

The function process_realtime_data feeds data into the real-time Model 3 and returns prediction values and feedback.

The parameter row is input data in the form of a series, and thus, for example, data may be input to the variable of inputs as described below.

inputs = [70, 30, 170, 50, 55, 30, 8, 10, 12, 100, 98, 60, 62, 90]

The inputs are converted into PyTorch tensors via torch.tensor, and these tensors are provided as inputs to the Model 3, which then feeds back outputs corresponding to the inputs.

The outputs fed back by the Model M3 are input to the above-described generate_feedback function along with the inputs above, and this function returns feedback corresponding to the inputs and outputs that are input.

Finally, the function process_realtime_data returns the outputs and the feedback, so that the patient performing real-time training may be provided with the necessary feedback.

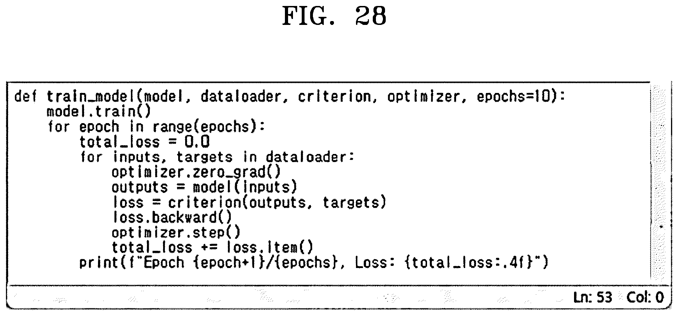

FIG. 28 is a code snippet of the function train_model for training the Model M3.

The **function train_model** is a function that implements a basic learning loop for training a model with given data. This function repeatedly updates the weights using the PyTorch model with training data and outputs the learning progress in epoch units.

Referring to FIG. 28, the model learning function train_model trains the model using the given data, the loss function, and the optimization algorithm, and its parameters are as follows.

- model: PyTorch model to train.
- dataloader: DataLoader of PyTorch that provides the training data set.
- criterion: The loss function
- optimizer: The optimization algorithm for weight updates
- epochs=10: The number of learning repetitions (a default value is 10)

This function implements a model training loop and processes data from the data set in units of batches and generates prediction values based on forward propagation through the model. The criterion is used as the loss function to calculate an error (loss) between the prediction value of the model and the actual value.

The loss value is converted into a gradient with respect to weights (parameters) of the model through the backpropagation algorithm, and the weights are updated through the optimization algorithm. Here, the learning status is monitored by calculating and outputting the cumulative loss value for one epoch (learning the entire data set once).

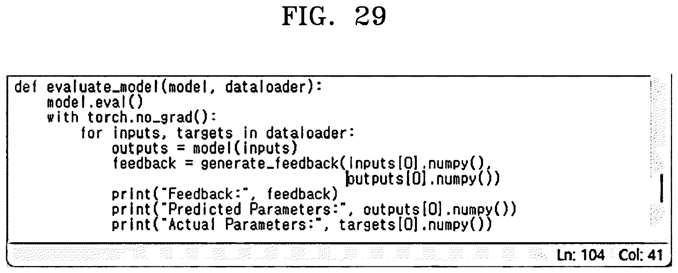

FIG. 29 is a code snippet of the evaluate_mode function that evaluates the trained model.

The **evaluate_model function** is a function for evaluating the performance of a trained model. It repeatedly processes data through a given data loader and compares a prediction value of the model with the actual value or generates feedback.

This process includes a procedure in which after the model is switched into the evaluation mode, the gradient calculation is inactivated, prediction values are generated by fetching inputs and actual values from the data loader in units of batches, feedback is generated based on the input value and the prediction value of the first sample, and the prediction value, the actual value, and the generated feedback are output.

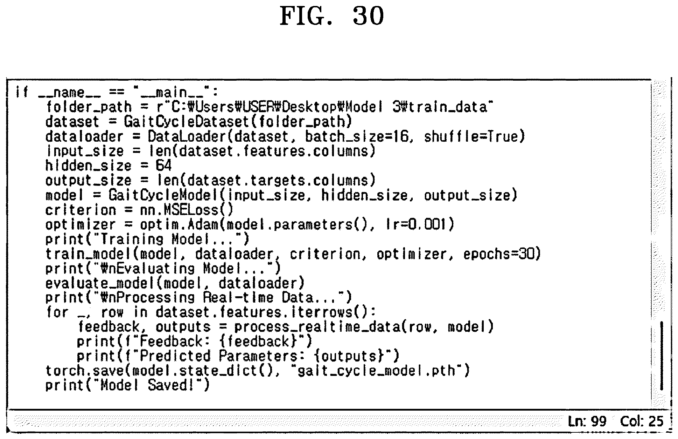

FIG. 30 is a snippet of an execution code for generating the Model 3 according to the present disclosure.

This execution code also loads the data set via the data loader and specifically includes the following processes.

- Load data set and create data loader
- Model initialization and training
- Evaluation of the trained model
- Real-time data processing and feedback provision
- Saving the trained Model M3

The Model M3 is initialized by the GaitCycleModel class and then trained by the train_model function. In this process, nn.MSELoss is used as a loss function to calculate the error between the prediction value and the actual value, and Adam is selected as the optimizer to update the weights.

The overall flow of the execution code derived for example is as follows.

The execution code uses the GaitCycleData set class to load data stored in the training data folder, initializes a model based on the data, and then performs the training, evaluation, real-time data processing, and model storage processes.

1. Data Preparation

The folder path storing the training data in the folder_path variable is specified, and the .csv file in that folder is read to create a data set by using the GaitCycleData set class. Thereafter, the data set is divided into 16 batch sizes and shuffled by using the DataLoader class of PyTorch to generate a DataLoader object to be used for training.

2. Model Initialization

The input data size (input_size) is set to the number of feature data columns of the data set, and the output data size (output_size) is set to the number of target data columns. Here, the hidden layer size (hidden_size) is set to 64, and the GaitCycleModel of the MLP structure is initialized based on this hidden layer size.

3. Setting Loss function and Optimizer

The loss function uses nn.MSELoss to calculate the MSE between the prediction value and the actual value. The optimizer is chosen as Adam, and the training rate (lr) is set to 0.001.

4. Training Model M3

After the message "Training Model..." is output, the train_model function is called to train the model for 30 epochs.

4. Evaluating Model M3

The message "Evaluating Model..." is output, and the evaluate_model function is called to evaluate the performance of the trained model.

5. Real-time Data Processing:

After the message "Processing Real-time Data..." is output, the feature data (data set.features) of the data set is iterated one row at a time and the process_realtime_data function is called.
The corresponding function generates feedback based on the input data and the output value of the model and outputs the generated feedback and the prediction value.

6. Saving Model:

The weight of the trained model is saved as a file of "gait_cycle_model.pth" by using torch.save. And then, the execution is terminated by outputting the message "Model Saved!"

IV. Model 4 (M4)

The Model 4 is an AI agent that evaluates training results and predicts future trends. Input data may be previous (past) training result data and current (real-time) training result data, and in this case, output data may be the trend analysis and future prediction based on past training results and the current training result data.
The training data may include data acquired in real time from a patient undergoing gait training. The data may include at least one of the following information: GRF (%), BWS (%), upper body inclination or tilting (degree), joint angle (degree), cadence, left gait cycle (0-99%), right gait cycle (0-99%), stride length, ankle angle, oxygen saturation data, and heart rate data.
Training parameters may include step length (cm), step height (cm), initial contact angle (degree), and toe off angle (degree).
This Model 4 may be an anomaly detection model based on time series information and may include, for example, an algorithm such as the LSTM, which is an extension type of the RNN and may learn long sequence data and handle long-term dependency, or the variational auto encoder (VAE), which is extended to appropriately process time series data.
This Model 4 is designed to analyze a current training result or compare the current training result with a previous training result to analyze the trend of results according to repeated training and predict future training based on this. This model may output graphs analyzing a previous training average trend line, a current training average trend line, and a future training predicted trend. To this end, trends of previous training results and current training results may be compared and analyzed, and future training performance may be predicted based on the trend of previous training results.
The Model 4 may be configured to include an anomaly detection model M4a and a trend prediction model M4b.

The anomaly detection model M4a detects abnormal patterns in real-time training data by utilizing algorithms such as an LSTM auto encoder or the VAE. This model detects abnormal data by setting a dynamic threshold value based on the reconstruction error (RE), thereby effectively analyzing the patient's current training status.

The trend prediction model M4b uses Seq2Seq-based LSTM/GRU or transformer-based models to predict, based on past and current data, the future training performance. This model samples time series data using a sliding window technique and analyzes multiple input features to learn correlations between data. Additionally, the two models work in conjunction with each other, and the results of the anomaly detection model M4a are reflected as weights for the trend prediction model M4b to improve prediction accuracy.

Output data of M4 may include visualization data in the form of graphs, results analysis data, comprehensive evaluation indicators, and improvement suggestion data.

- Visualization data in graph form: A previous training average trend line, a current training average trend line, and a future training performance prediction trend line are included, through which a user may intuitively understand training performance changes over time.
- Results analysis data: The difference between the previous and current training is analyzed to provide improvement or decrease rates in key indicators. Also, by highlighting anomalous data detected in specific sections, problems that occurred during training may be clearly identified.
- Comprehensive evaluation indicators and improvement suggestion data: The comprehensive evaluation indicators evaluate the patient's performance on a scale of 0 to 100 grades and suggests specific adjustment recommendations for training parameters that require improvement (e.g. step length, step height, etc.).

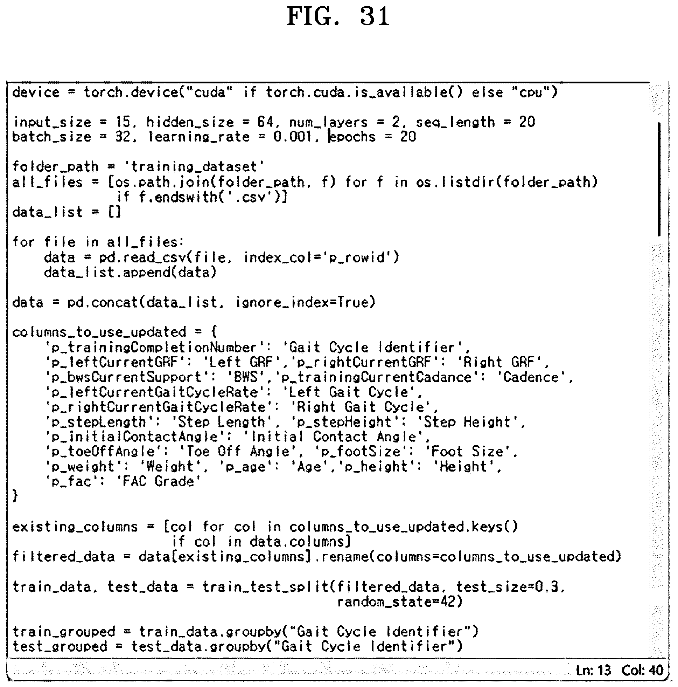

FIG. 31 shows a code snippet of a data preprocessing and training pipeline utilizing the LSTM Model M4 for anomaly detection and trend prediction, according to the present disclosure.

The program for training the Model M4 is written in Python, for example, and a number of libraries may be imported as follows.

The followings may be imported: the OS module for interacting with the operating system, pandas as pd for data analysis and processing, torch, which is the core module of the PyTorch framework, torch.nn, which provides neural network components, the torch.optim module, which provides optimization algorithms for training deep learning models, and torch.utils.data, which provides tools for efficiently managing data and processing the data in units of batches.

Hyper-parameters may be set for example as follows.

The number of features in input data: input_size = 15:

The LSTM hidden layer size: hidden_size = 64
The number of layers in LSTM: num_layers = 2
The sequence length: seq_length = 20
The batch size: batch_size = 32
The learning rate: learning_rate = 0.001
The number of epochs: epochs = 20

The pipeline for processing data may include the following processes.

1. Data loading and preprocessing

In this process, all CSV files stored in a specified folder, for example, the "training_data set" folder, are read and merged into a single Pandas DataFrame, and the process includes filtering the .csv files in the training_data set folder, reading data files and collecting data, and integrating (merging) data of the data files into one so as to form a data frame used for analyzing and processing.

- Forming a dictionary (columns_to_use_updated) of the data structure

The dictionary stores data as a pair of an input key and an output value, so as to obtain the name to be used for actual output as an output value through the input key.

```
columns_to_use_updated = {
    'p_trainingCompletionNumber': 'Gait Cycle Identifier,'
    'p_leftCurrentGRF': 'Left GRF,'
    'p_rightCurrentGRF': 'Right GRF,'
    'p_bwsCurrentSupport': 'BWS,'
    'p_trainingCurrentCadance': 'Cadence,'
```

```
'p_leftCurrentGaitCycleRate': 'Left Gait Cycle,'
'p_rightCurrentGaitCycleRate': 'Right Gait Cycle,'
'p_stepLength': 'Step Length,'
'p_stepHeight': 'Step Height,'
'p_initialContactAngle': 'Initial Contact Angle,'
'p_toeOffAngle': 'Toe Off Angle,'
'p_footSize': 'Foot Size,'
'p_weight': 'Weight,'
'p_age': 'Age,'
'p_height': 'Height,'
'p_fac': 'FAC Grade'
}
```

2. Data separation:

In this operation, some of the selected data filtered_data from the entire data is divided into training data train_data and test data test_data. In the example code below, it is divided in a ratio of 7:3.

```
train_data, test_data = train_test_split(filtered_data, test_size=0.3, random_state=42)
```

3. Grouping:

Data is grouped based on the "Gait Cycle Indentifier," which is the output value of the dictionary.

```
train_grouped = train_data.groupby ("Gait Cycle Identifier")
test_grouped = test_data.groupby("Gait Cycle Identifier")
```

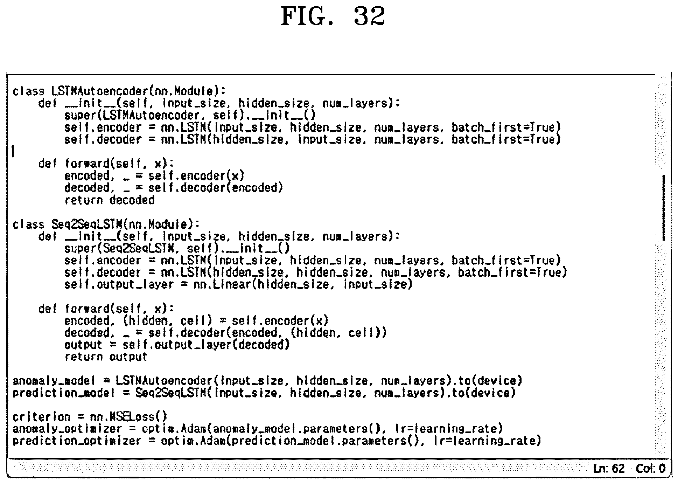

FIG. 32 is a code snippet illustrating the declaration of an LSTM-based auto encoder class and a Seq2Seq class for anomaly detection and trend prediction and an initialization process of two models utilizing these classes, according to the present disclosure.

The initialized anomaly detection model M4a is anomaly_model, and the trend prediction model M4b is prediction_model. The loss function for each model uses the MSE, and Adam is applied as the optimization algorithm to update the learning parameters of each model.

The code snippet below may contain a series of command statements that train and evaluate the anomaly detection model M4a and trend prediction model M4b of the M4 model and visualize the results.

```
train_anomaly_model (train_grouped, epochs)
train_prediction_model (train_grouped, epochs)
evaluate_anomaly_model (test_grouped)
visualize_analyze_and_suggest_results (test_grouped)
```

As described above, the functions train_anomaly_model and train_prediction_model train the anomaly detection model and trend prediction model respectively, the function evaluate_anomaly_model evaluates the performance of the anomaly detection model, and the function visualize_analyze_and_suggest_results visualizes and analyzes the test results and suggests improvement suggestions.

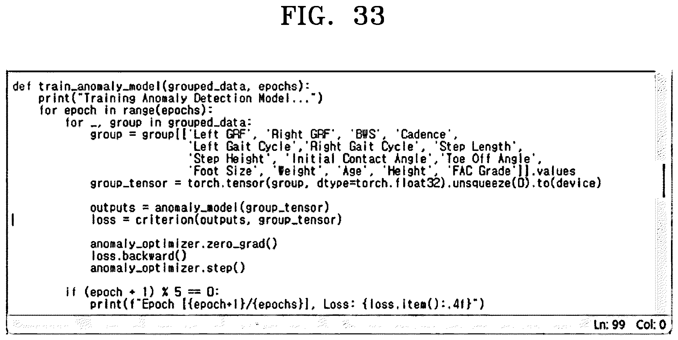

FIG. 33 illustrates a definition of the class of a function train_anomaly_model for training the anomaly detection model M4a described above, which is one element of the M4 model according to the present disclosure.

Referring to FIG. 33, after the model M4a learns the pattern of normal data, the model M4a detects abnormal behavior exhibiting high loss for abnormal data, and the model M4a is trained through the following process.

- Data preprocessing: Separating data into groups and converting the data into a tensor format
- Model training: Inputting data into a model to generate a prediction value, calculating the loss between the prediction value and the actual value, and updating the model weights through backpropagation.
- Process outputting: Monitoring the learning process by outputting the loss value every 5 epochs.

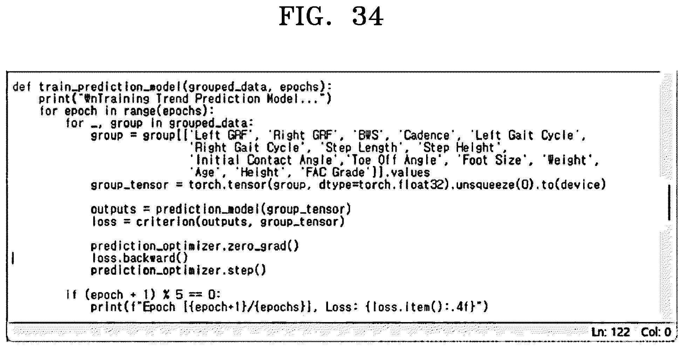

FIG. 34 illustrates the definition of a function for training the trend prediction model M4b in the model M4 according to the present disclosure.

Referring to FIG. 34, the training of the model M4b includes the following processes.

- Training by dividing data into grouped units
- Converting each group data into a tensor and passing it to the model to generate a prediction value
- Calculating the loss between predicted and actual values

- Calculating the gradient according to the loss through backpropagation and updating the model weights based on this
- Monitoring the training progress by outputting a loss value every 5 epochs

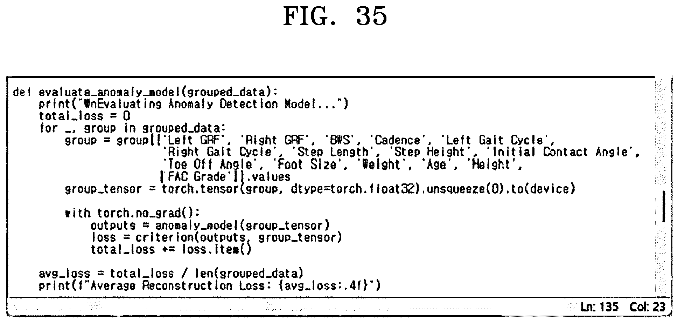

FIG. 35 illustrates the definition of a function evaluate_anomaly_model for evaluating the anomaly detection model M4a in the M4 model according to the present disclosure.

This function includes the following evaluation processes of M4a.

- Data preparation: Processing data in group units and moving the processed data to evaluation device
- Performing model reconstruction: Reconstructing input data and generating an output value, via the model
- Calculating reconstruction loss: Calculating and saving the loss between the input data and the output value
- Outputting average loss: Calculating and outputting the average reconstruction loss for all group data.

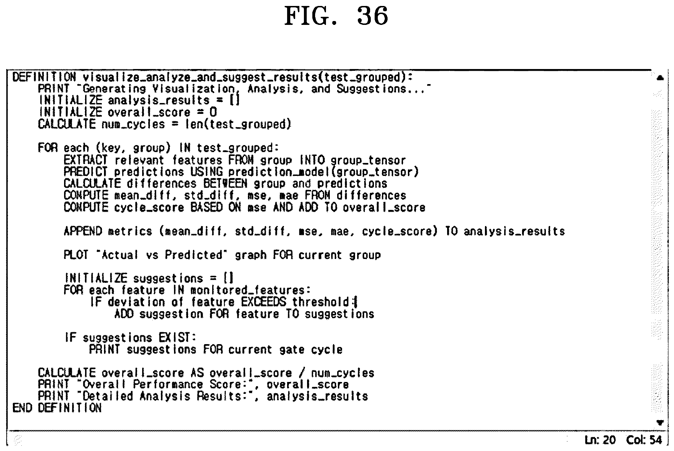

FIG. 36 is a pseudo code snippet of the function visualize_analyze_and_suggest_results for visualizing and analyzing test results and suggesting improvement suggestions in the M4 model according to the present disclosure. Some pseudocodes in FIG. 36 may be specified as follows.

- INITIALIZE analysis_results = [], overall_score = 0, num_cycles = len (test_grouped):

```
analysis_results = []
overall_score = 0
num_cycles = len (test_grouped)
```

The code initializes the list to store the analysis results (analysis_results), the total score (overall_score), and the number of groups of test data (num_cycles).

- EXTRACT relevant features FROM group INTO group_tensor:
  group = group[['Left GRF', 'Right GRF', 'BWS', ...]].values group_tensor = torch.tensor(group, dtype=torch.float-t32).unsqueeze(0).to(device)

The code above filters the group data to extract (group) only the necessary features and converts the features to a PyTorch tensor (group_tensor).

- PREDICT predictions USING prediction_model(group_tensor):

```
with torch.no_grad():
    predictions = prediction_model(group_tensor).squeeze(0).cpu().numpy()
```

The code above uses a trend prediction model (prediction_model) to predict future values for the current group.

- COMPUTE mean_diff, std_diff, mse, mae FROM differences:

```
mean_diff = np.mean(differences, axis=0)
std_diff = np.std(differences, axis=0)
mse = np.mean(np.square(differences), axis=0)
mae = np.mean(np.abs(differences), axis=0)
```

The code above calculates the mean difference (mean_diff), standard deviation (std_diff), mean square error (mse), and mean absolute error (mae) based on the differences.

- COMPUTE cycle_score BASED ON mse AND ADD TO overall_score:

$$cycle\_score = max(0, 100 - (np.mean(mse) * 10))$$

$$overall\_score \mathrel{+}= cycle\_score$$

The code above calculates the score (cycle_score) of the current group and adds the score to the overall score (overall_score).

- APPEND metrics TO analysis_results:

```
analysis_results.append({ "Gait Cycle": key, "Mean Difference":
 mean_diff.tolist(), ...})
```

The code above saves the computed metrics (mean difference, standard deviation, loss value, etc.) to the analysis results list (analysis_results).

- PLOT "Actual vs Predicted" graph FOR current group:

```
plt.figure(figsize=(10, 6))
for i, feature in enumerate(['Left GRF', ...]):
  plt.plot(group[:, i], label=f"Actual {feature}")
  plt.plot(predictions[:, i], linestyle='--', label=f"Predicted {feature}")
plt.show()
```

The code above visualizes a graph comparing the actual value and the prediction value with respect to the current group.

- INITIALIZE suggestions = [] AND CHECK deviation FOR monitored_features:

```
suggestions = []
    for i, feature in enumerate(['Step Length', ...]):
        if np.abs(mean_diff[i]) > 0.1 * np.mean(group[:, i]):
            suggestions.append(f"Consider adjusting {feature}. Current deviation:
            {mean_diff[i]:.2f}")
```

The code above generates suggestions when the deviation of a specific feature (step length, step height, etc.) exceeds a threshold value.

- PRINT overall_score AND analysis_results:

```
print(f"Overall Performance Score (0-100): {overall_score:.2f}")

print("\nDetailed Analysis Results:")
```

The code above outputs the overall performance score and detailed analysis results.

As described above, in the Model 4 M4 according to the present disclosure, the anomaly detection model M4a evaluates the extent to which the model has learned normal data patterns through reconstruction loss. A low reconstruction loss indicates that the anomaly detection model has learned the normal patterns in the data well, while a decreasing loss over epochs indicates that the anomaly detection model is performing progressively better. The trend prediction model M4b described above indicates how accurately the model predicted the future based on past data through prediction loss. The lower loss per epoch shows that the trend prediction model has learned the correlation between data well.

The evaluation of these models M4a and M4b uses average reconstruction loss, cycle score, and graphical visual comparison.

The average reconstruction loss is computed on the test data, and a lower value indicates that the model performs better at reconstructing normal data.

The cycle score is an indicator of the performance of the gait cycle on a scale of 0 to 100 points. A low cycle score may indicate that the prediction performance is low, the prediction performance indicating how accurately the gait cycle may be predicted, or that data may include outliers, and the cycle score may help identify problematic sections of a particular cycle.

The visual comparison may be made by overlaying the actual and prediction values for each gait cycle on a single display, with each distinguishable line (e.g., a dotted line and a solid line) representing each of the actual value and the prediction value. In this case, the better the line representing the actual value and the line representing the prediction value overlap, the better the performance of the model is. When a large deviation appears in a specific characteristic, additional model adjustment may be required for that characteristic.

The results analysis comprehensively evaluates the performance of the model, including the mean difference between the actual value and the prediction value of each feature (the lower the value, the better the prediction accuracy), the MSE and MAE that evaluate the prediction error (the lower the value, the better the performance), and the standard deviation that indicates the variability of the prediction error (the lower the value, the more stable the performance).

V. Model 5 (M5)

Model 5 is an AI model that supports smart training result reporting. The input data includes pre- and post-training result data and report generation-type, and the output includes text or images, such as images and videos.

The input report generation-type is simple or multimodal support generative pre-trained transformer GPT model-based prompt input. Also, the output includes specifically, for example, text in a PDF document format, images with respect to training results and status for each patient (with different backgrounds and progress states), or videos of walking trajectories for each patient. Another output includes a video-based output based on the patient gait training analysis results, such as virtual avatar movement, self-figure movement, and video output in the form of a butterfly diagram.

In gait rehabilitation training systems, the "butterfly diagram" is a visual tool primarily used for gait analysis and evaluation. This diagram helps to analyze the gait cycle, such as footprint analysis, gait cycle evaluation, rehabilitation effect measurement, left-right asymmetry analysis of gait, etc., by visualizing the pattern and pressure of the foot contacting the ground. Such diagrams are usually named "butterfly" because the shape of the footprint and the pressure distribution resemble the shape of the butterfly's wings, and a butterfly diagram is illustrated in FIG. 23.

In the case of the multimodal support GPT, it will be possible to induce prompt-based reporting (text, image, video) for the work generated above.

Also, in addition to the output described above, it is possible to enable tasks such as training and report outputting through voice outputs. By doing so, it may be expected to provide patients with further intuitive and effective training results and move in a direction toward increased training achievement and satisfaction through smart reporting support on the patient's condition and training results after training.

Hereinafter, example configurations of the Model 5 according to the present disclosure are described.

Input data for AI agents applying the Model 5 may include pre- and post-training result data, a report generation type, and a prompt input.

As the pre- and post-training result data, past and present training results are included, and training parameters and real-time data are used in an integrated manner. Primary data items may include, as described above, primary data items, such as step length, step height, GRF, BWS, ankle angle, gait cycle, etc.

The output of the Model 5, i.e. the report generation type, may include a basic report that provides a simple text or image-based summary of results and a multimodal report containing text, images, and videos generated with GPT-based prompted inputs, and may diversify the data analysis and visualization method depending on the user selection type.

Prompts for creating GPT-based reports based on a prompt input for multimodal reports. For example, it may be provided as "please visualize the patient's walking trajectory and output it as a video."

The Model 5 (M5) above may include a report automation model, a video-based analysis model, and a gait evaluation index prediction model.

- Report Automation Model:

Input data is analyzed by using a multimodal support GPT-based model, and training results are automatically generated in the form of text, images, and videos. This model may understand training data and produce reports in an appropriate format based on given prompts.

- Video-based Analysis Model

This model analyzes and visualizes the patient's walking trajectory based on time series data and may, for example, visualize the gait patterns, such as movement simulation of a virtual avatar, the patient's actual shape movement, or "the butterfly diagram."

- Gait Evaluation Index Prediction Model

This model applies a deep learning model based on LSTM and Transformer to predict gait evaluation indices, automatically establishes a training plan based on the prediction results, and derives improvement points.

The output of the Model 5 (M5) above may include: training results in a report form; real-time voice outputs; training results visualized based on videos; and smart reporting.

The training results in the form of the report may include a text report in the form of a PDF document containing a summary of training performance and analysis results of key indicators, an image report containing visual data reflecting the patient's various backgrounds and progress states, and a video report presenting a gait trajectory simulation, for example, the gait movement of the patient's own figure or a virtual avatar, a butterfly diagram, etc.

As the real-time voice output, the training progress and results may be provided as real-time voice guidance. For example, current training status information, "The current walking cycle average speed is 90% of the target speed," may be provided.

The video-based visualized training results may include video-enhanced results using a virtual avatar or self-image and visual presentation of gait cycle asymmetry and rehabilitation effects using "butterfly diagrams."

The smart reporting may improve patient training achievement and satisfaction by suggesting personalized training performance summaries and improvement directions based on the patient's condition and training results and providing intuitive and easy-to-understand reporting materials.

According to the gait training system and method of the present disclosure applying the aforementioned multiple stages of AI agents, i.e., the Models 1 to 5 (M1, M2, M3, M4, and M5), it is possible to establish a patient-friendly gait training plan and to efficiently perform personalized and patient-friendly rehabilitation training for the patient. According to the rehabilitation training according to the system and the method, gait evaluation indices may be accurately predicted using AI models at each stage, training plans may be automatically established based on these, and training results may be further accurately evaluated.

According to the rehabilitation training according to the system and the method, gait evaluation indices may be accurately predicted using AI models at each stage, training plans may be automatically established based on these, and training results may be further accurately evaluated. Therefore, the intervention of therapists according to the prescription of medical staff may be reduced compared to existing methods and systems.

While the exemplary embodiments of the present disclosure have been described in detail above according to exemplary embodiments, it will be apparent to one of ordinary skill in the art that various modifications may be made and practiced in the present disclosure without departing from the spirit and scope of the present disclosure as defined by the appended claims. Therefore, modifications of future embodiments of the present disclosure shall not deviate from the technology of the present disclosure.

**Claims**

**1.** An artificial intelligence (AI)-based robot-assisted orthopedic exercise system comprising: a gait training apparatus comprising a pedal on which a patient stands and a pedal driver providing one or more links that drive the pedal; and a system control unit configured to control the pedal driver according to a training plan established for each individual to perform gait training of the patient standing on the pedal,
wherein the system control unit comprises:

an index prediction unit configured to determine a gait evaluation index of the patient, based on patient's initial gait status information or information about a gait training result after initial gait training;
a training planning unit configured to establish, based on the gait training result, an individual-tailored training plan of the patient, performed by the gait training apparatus;
a training progress unit configured to perform training on the patient according to the training plan, monitor, in real time, training data with respect to a real-time training status from the patient, and generate feedback with respect to the training data; and
a training evaluation unit comprising a training evaluation model configured to evaluate a training result, based on training result data according to results of rehabilitation training of the patient performed by the gait training apparatus.

**2.** The AI-based robot-assisted orthopedic exercise system of claim 1, wherein the training data comprises information about at least one characteristic from among a ground reaction force (GRF) (%), a body weight support (BWS) (%), an upper body inclination or tilting (degree), a joint angle (degree), a cadence, a left gait cycle (%), a right gait cycle (%), a stride length, an ankle angle, oxygen saturation data, and heart rate data.

**3.** The AI-based robot-assisted orthopedic exercise system of claim 1, wherein the training evaluation model is configured to provide an anomaly detection model and a trend prediction model, and
the anomaly detection model detects abnormal patterns in real-time training data, and the trend prediction model predicts future training performance based on past training data and current training data.

**4.** The AI-based robot-assisted orthopedic exercise system of claim 3, wherein the anomaly detection model detects the abnormal patterns in the real-time training data, based on at least one of a long short-term memory (LSTM) autoencoder or a variational autoencoder (VAE), and
the trend prediction model predicts the future training performance, based on past data and current data, by utilizing a Seq2Seq-based LSTM/gated recurrent unit (GRU) or transformer-based model.

**5.** The AI-based robot-assisted orthopedic exercise system of claim 1, wherein the training progress unit comprises a training progress model configured to generate the feedback on the training data, and
the training progress model is configured to receive at least one training parameter from among a step length (cm), a step height (cm), an initial contact angle (degree), and a toe off angle (degree) and output, based on the training data, a correction value for the at least one training parameter.

**6.** The AI-based robot-assisted orthopedic exercise system of claim 5, wherein the training progress model comprises any one deep learning model from a multi-layer perceptron (MLP) and an open vision-language model (VLM).

**7.** The AI-based robot-assisted orthopedic exercise system of claim 1, wherein image information or voice information of the patient undergoing training or a therapist assisting the patient's training is obtained and used as input data of the training progress model, and the training progress model is configured to generate output data corresponding to the input data.

**8.** The AI-based robot-assisted orthopedic exercise system of claim 1, wherein the training planning unit further comprises a reference training planning model trained using patient information, training protocols, training time, training parameters, etc. set by a medical professional, and the training planning unit is configured to output a final training plan that is corrected or compensated based on outputs of an autonomous training planning model and the reference training planning model.

**9.** The AI-based robot-assisted orthopedic exercise system of claim 8, wherein the training planning unit further comprises a final training planning model configured to output the final training plan based on the outputs of the autonomous training planning model and the reference training planning model.

**10.** The AI-based robot-assisted orthopedic exercise system of claim 1, further comprising a monitoring unit configured to monitor a status of the rehabilitation training performed by the gait training apparatus according to the training plan, obtain information during the training, and generate therefrom training status information with respect to the patient's gait training.

**11.** A method of providing an artificial intelligence (AI)-based robot-assisted orthopedic exercise, the method comprising: predicting, by an index prediction unit, a gait evaluation index of a patient, based on initial gait status information or information about a gait training result after initial gait training;

establishing, by a training planning unit, an individual-tailored training plan of the patient, by using an autonomous training planning model, based on the gait evaluation index;
while a gait training apparatus comprising an end-effector-type pedal and a pedal driver providing one or more links that drive the pedal, is performing rehabilitation training of the patient according to the training plan, monitoring, by a training progress unit, in real time, training data with respect to a real-time training status from the patient and generating feedback with respect to the training data; and
evaluating, by a training evaluation unit, the training results, based on training result data according to training results.

**12.** The method of claim 11, wherein the training data comprises at least one from among a ground reaction force (GRF), a body weight support (BWS), an upper body inclination or tilting, a joint angle, a cadence, a left gait cycle, a right gait cycle, a stride length, an ankle angle, oxygen saturation data, and heart rate data.

**13.** The method of claim 11, wherein the training evaluation unit comprises a training evaluation model configured to evaluate the training result, based on the training result data based on results of the rehabilitation training of the patient performed by the gait training apparatus,

the training evaluation model is configured to provide an anomaly detection model and a trend prediction model, and
the anomaly detection model detects abnormal patterns in real-time training data, and the trend prediction model predicts future training performance based on past training data and current training data.

**14.** The method of claim 13, wherein the anomaly detection model detects the abnormal patterns in the real-time training data, based on at least one of a long short-term memory (LSTM) autoencoder or a variational autoencoder (VAE), and
the trend prediction model predicts the future training performance, based on past data and current data, by utilizing a Seq2Seq-based LSTM/gated recurrent unit (GRU) or transformer-based model.

**15.** The method of claim 11, wherein the training progress unit comprises a training progress model configured to generate the feedback on the training data, and
the training progress model is configured to receive at least one training parameter from among a step length (cm), a step height (cm), an initial contact angle (degree), and a toe off angle (degree) and output, based on the training data, a correction value for the at least one training parameter.

**16.** The method of claim 15, wherein the training progress model comprises any one deep learning model from a multi-layer perceptron (MLP) and an open vision-language model (VLM).

**17.** The method of claim 11, wherein an image or voice recording device is configured to obtain image information or voice information of the patient undergoing training or a therapist assisting the patient's training,

an analysis device is configured to analyze the image information or the voice information, and
the training progress model is configured to use input data of the training progress model and generate output data corresponding to the input data.

**18.** The method of claim 11, wherein the training planning unit further comprises:

the autonomous training planning model that establishes the individual-tailored training plan of the trained patient according to the initial gait status information or the gait training result after the gait training; and
a reference training planning model trained using patient information, training protocols, training time, training parameters, etc. set by a medical professional, and
the training planning unit is configured to output a final training plan that is corrected or compensated based on outputs of the autonomous training planning model and the reference training planning model.

**19.** The method of claim 11, wherein a training-status detection unit detects, as characteristics information during training, information about at least one of a GRF, a BWS, an upper body inclination or tilting, a joint angle, a cadence, a left gait cycle, a right gait cycle, a stride length, an ankle angle, oxygen saturation data, and heart rate data of the patient undergoing the gait training.

**20.** The method of claim 11, wherein a monitoring unit is configured to monitor a status of the rehabilitation training performed by the gait training apparatus according to the training plan, obtain information during the training, and generate therefrom training status information with respect to the patient's gait training.

**1.** An artificial intelligence (AI)-based robot-assisted orthopedic exercise system comprising: a gait training apparatus comprising a pedal on which a patient stands; and a system control unit configured to control the gait training apparatus to perform gait training of the patient standing on the pedal,
wherein the gait training apparatus comprises:

a reciprocating movement driving device configured to perform a linear reciprocating motion with respect to a main body base of the exercise system;
an operating link coupled to the reciprocating movement driving device to be capable of a rotary motion according to a first driving device; and
a second driving device coupled to the operating link to be capable of rotating the pedal, and
the system control unit comprises:

an index prediction unit comprising an index prediction model configured to determine a gait evaluation index of the patient, based on patient's initial gait status information or information about a gait training result after initial gait training;
a training planning unit comprising a reference training planning model trained based on patient information, training protocols, training time, and training parameters set by a medical professional and an autonomous training planning model configured to establish, based on information about the gait training result, an individual-tailored training plan of the patient, performed by the gait training apparatus;

a training progress unit comprising a training progress model configured to perform training on the patient according to the training plan, monitor, in real time, training data with respect to a real-time training status from the patient, and generate feedback with respect to the training data; and

a training evaluation unit configured to evaluate a training result, based on training result data according to results of rehabilitation training of the patient performed by the gait training apparatus, the training evaluation unit comprising an anomaly detection model and a trend prediction model.

**2.** The AI-based robot-assisted orthopedic exercise system of claim 1, wherein the training data comprises information about at least one characteristic from among a ground reaction force (GRF) (%), a body weight support (BWS) (%), an upper body inclination or tilting (degree), a joint angle (degree), a cadence, a left gait cycle (%), a right gait cycle (%), a stride length, an ankle angle, oxygen saturation data, and heart rate data.

**3.** The AI-based robot-assisted orthopedic exercise system of claim 1, wherein the anomaly detection model detects abnormal patterns in real-time training data, and the trend prediction model predicts future training performance based on past training data and current training data.

**4.** The AI-based robot-assisted orthopedic exercise system of claim 3, wherein the anomaly detection model detects the abnormal patterns in the real-time training data, based on at least one of a long short-term memory (LSTM) autoencoder or a variational autoencoder (VAE), and the trend prediction model predicts the future training performance, based on past data and current data, by utilizing a Seq2Seq-based LSTM/gated recurrent unit (GRU) or transformer-based model.

**5.** The AI-based robot-assisted orthopedic exercise system of claim 1, wherein the training progress model is configured to receive at least one training parameter from among a step length (cm), a step height (cm), an initial contact angle (degree), and a toe off angle (degree) and output, based on the training data, a correction value for the at least one training parameter.

**6.** The AI-based robot-assisted orthopedic exercise system of claim 5, wherein the training progress model comprises any one deep learning model from a multi-layer perceptron (MLP) and an open vision-language model (VLM).

**7.** The AI-based robot-assisted orthopedic exercise system of claim 1, wherein image information or voice information of the patient undergoing training or a therapist assisting the patient's training is obtained and used as input data of the training progress model, and the training progress model is configured to generate output data corresponding to the input data.

**8.** The AI-based robot-assisted orthopedic exercise system of claim 1, wherein the training planning unit is configured to output a final training plan that is corrected or compensated based on outputs of an autonomous training planning model and the reference training planning model.

**9.** The AI-based robot-assisted orthopedic exercise system of claim 8, wherein the training planning unit further comprises a final training planning model configured to output the final training plan based on the outputs of the autonomous training planning model and the reference training planning model.

**10.** The AI-based robot-assisted orthopedic exercise system of claim 1, further comprising a monitoring unit configured to monitor a status of the rehabilitation training performed by the gait training apparatus according to the training plan, obtain information during the training, and generate therefrom training status information with respect to the patient's gait training.

**11.** A method of controlling an artificial intelligence (AI)-based robot-assisted orthopedic exercise system controlling an exercise system comprising a gait training apparatus comprising right and left pedals on which a patient stands and a system control unit configured to control the gait training apparatus to perform gait training of the patient standing on the right and left pedals,
wherein the gait training apparatus comprises:

a reciprocating movement driving device configured to perform a linear reciprocating motion with respect to a main body base of the exercise system;
an operating link coupled to the reciprocating movement driving device to be capable of a rotary motion according to a first driving device; and

a second driving device coupled to the operating link to be capable of rotating the pedals, the method comprising:

predicting, by an index prediction unit, by using an index prediction model, a gait evaluation index of a patient, based on initial gait status information or information about a gait training result after initial gait training;

establishing, by a training planning unit, an individual-tailored training plan of the patient, by using a reference training planning model trained based on patient information, training protocols, training time, and training parameters set by a medical professional and an autonomous training planning model based on the gait evaluation index;

while a gait training apparatus comprising an end-effector-type pedal and a pedal driver providing one or more links that drive the pedal, is performing rehabilitation training of the patient according to the training plan, monitoring, by a training progress unit, in real time, training data with respect to a real-time training status from the patient and generating feedback with respect to the training data by using a training progress model configured to generate the feedback on the training data ; and

evaluating, by a training evaluation unit, by using an anomaly detection model and a trend prediction model, the training results, based on training result data according to training results.

**12.** The method of claim 11, wherein the training data comprises at least one from among a ground reaction force (GRF), a body weight support (BWS), an upper body inclination or tilting, a joint angle, a cadence, a left gait cycle, a right gait cycle, a stride length, an ankle angle, oxygen saturation data, and heart rate data.

**13.** The method of claim 11, wherein the anomaly detection model detects abnormal patterns in real-time training data, and the trend prediction model predicts future training performance based on past training data and current training data.

**14.** The method of claim 13, wherein the anomaly detection model detects the abnormal patterns in the real-time training data, based on at least one of a long short-term memory (LSTM) autoencoder or a variational autoencoder (VAE), and
the trend prediction model predicts the future training performance, based on past data and current data, by utilizing a Seq2Seq-based LSTM/gated recurrent unit (GRU) or transformer-based model.

**15.** The method of claim 11, wherein the training progress model is configured to receive at least one training parameter from among a step length (cm), a step height (cm), an initial contact angle (degree), and a toe off angle (degree) and output, based on the training data, a correction value for the at least one training parameter.

**16.** The method of claim 11, wherein the training progress model comprises any one deep learning model from a multi-layer perceptron (MLP) and an open vision-language model (VLM).

**17.** The method of claim 11, wherein an image or voice recording device is configured to obtain image information or voice information of the patient undergoing training or a therapist assisting the patient's training, an analysis device is configured to analyze the image information or the voice information, and
the image information or the voice information is used as input data of the training progress model to generate output data corresponding to the input data.

**18.** The method of claim 11, wherein the training planning unit is configured to output a final training plan that is corrected or compensated based on outputs of the autonomous training planning model and the reference training planning model.

**19.** The method of claim 11, wherein a training-status detection unit detects, as characteristics information during training, information about at least one of a GRF, a BWS, an upper body inclination or tilting, a joint angle, a cadence, a left gait cycle, a right gait cycle, a stride length, an ankle angle, oxygen saturation data, and heart rate data of the patient undergoing the gait training.

**20.** The method of claim 11, wherein a monitoring unit is configured to monitor a status of the rehabilitation training performed by the gait training apparatus according to the training plan, obtain information during the training, and generate therefrom training status information with respect to the patient's gait training.

# FIG. 1

100

STATUS MONITOR — 113(102)

115

HMD

111

SYSTEM OPERATION DEVICE

CONTROL MONITOR — 110

KEYBOARD — 114

CONTROL | FEEDBACK

DETECTION UNIT — 105e

GAIT TRAINING APPARATUS — 105

# FIG. 2

# FIG. 3

# FIG. 4

# FIG. 5

# FIG. 6

# FIG. 7

| EVENTS | Initial contact | Opposite toe off | | Heel rise | | Opposite Initial contact | Toe off | Feet adjacent | | Tibia vertical | Next initial contact |
|---|---|---|---|---|---|---|---|---|---|---|---|
| | 0% | 10% | | 30% | | 50% | 60% | 73% | | 87% | 100% |
| PERIODS | Loading response | Mid stance | | Terminal stance | | | Pre swing | Initial swing | Mid swing | | Terminal swing |
| TASKS | Weight acceptance | Single-limb support | | | | | | Limb advancement | | | |
| PHASES | Stance phase | | | | | | | Swing phase | | | |
| CYCLE | Right gait cycle | | | | | | | | | | |

EP 4 759 288 A1

# FIG. 8

STRIDE LENGTH(Step)  STRIDE LENGTH(Step)

STRIDE WIDTH
(Step Width)

STRIDE (stride)

# FIG. 9

| SYSTEM CONTROL UNIT | ~111 |
|---|---|

| INPUT UNIT | ~111a |
|---|---|

| INDEX PREDICTION UNIT (M1) | ~111b |
|---|---|

| TRAINING PLANNING UNIT (M2) | ~111c |
|---|---|

| TRAINING PROGRESS UNIT (M3) | ~111d |
|---|---|

| TRAINING EVALUATION UNIT (M4) | ~111e |
|---|---|

| REPORTING UNIT (M5) | ~111f |
|---|---|

# FIG. 10

DEFAULT INPUT DATA

DATA SET INCLUDING PATIENT'S PERSONAL,
PHYSICAL, AND SYMPTOM INFORMATION
AND GAIT EVALUATION INDEX INFORMATION

TRAINING RESULT TEST DATA SET (CSV)

OPTIONAL INPUT DATA

TRAINING RESULT DATA SET (IMAGE)

TRAINING RESULT DATA SET (TEXT)

TRAINING RESULT DATA SET (MOV)

# FIG. 11

```
        ┌──────────┐
        │  START   │
        └──────────┘
              │
              ▼
         ╱─────────╲        N
        ╱  NEW      ╲──────────────────────────────────┐
        ╲  PATIENT? ╱                                   │
         ╲─────────╱                                    │
              │ Y                                        │
              ▼                                          ▼
    ┌────────────────────┐  S11    ┌──────────────────────────────────────┐
    │  INPUT TEST        │         │ INPUT AND VERIFY PERSONAL INFORMATION │
    │  DATA SET          │         │ CALL PREVIOUS GAIT EVALUATION INDEX   │
    └────────────────────┘         └──────────────────────────────────────┘
              │            S12                          │
              ▼                                          │
    ┌────────────────────┐                               │
    │ GENERATE INITIAL   │                               │
    │ GAIT EVALUATION    │                               │
    │ INDEX              │                               │
    └────────────────────┘                               │
              │                                          │
              └──────────────────┐          ┌────────────┘
                                 ▼          ▼
                        ┌──────────────────────────┐ S13
                        │   GENERATE AND           │
                        │   UPDATE TRAINING PLAN   │
                        └──────────────────────────┘
         ╱─────────╲ Y                   │
        ╱  UPDATE?  ╲◄──────┐            ▼
        ╲           ╱   N   │   ┌──────────────────────────┐ S14
         ╲─────────╱────────┘   │  PERFORM GAIT TRAINING   │
              ▲                 └──────────────────────────┘
              │                            │
              │                            ▼
              │                 ┌──────────────────────────┐ S15
              │                 │ GENERATE INTERMEDIATE    │
              │                 │ GAIT EVALUATION INDEX    │
              │                 └──────────────────────────┘
              │                            │
              │                            ▼
              │                 ┌──────────────────────────┐ S16
              │                 │  ANALYZE TRAINING RESULT │
              │                 └──────────────────────────┘
              │                            │
              │                            ▼
              │                 ┌──────────────────────────┐ S17
              │                 │ GENERATE MULTI-MODAL     │
              │                 │ REPORT                   │
              │                 └──────────────────────────┘
              │                            │
              │ Y             ╱─────────────────╲
              └──────────────╱  CONTINUE        ╲
                             ╲  TRAINING?        ╱
                              ╲─────────────────╱
                                       │ N
                                       ▼
                                ┌──────────┐
                                │   END    │
                                └──────────┘
```

# FIG. 12

START

INPUT TEST DATA SET — S11

GENERATE INITIAL GAIT EVALUATION INDEX — S12

GAIT EVALUATION INDEX PREDICTION MODEL — M1

GENERATE AND UPDATE TRAINING PLAN — S13

TRAINING PLAN GENERATION MODEL — M2

UPDATE?

Y

N

PERFORM GAIT TRAINING — S14

MONITORING MODEL (CHATBOT) — M3

GENERATE INTERMEDIATE GAIT EVALUATION INDEX — S15

GAIT EVALUATION INDEX PREDICTION MODEL — M1

GENERATE INITIAL GAIT EVALUATION INDEX — S16

TRAINING ANALYSIS MODEL — M4

GENERATE INITIAL GAIT EVALUATION INDEX — S17

REPORT GENERATION MODEL — M5

CONTINUE TRAINING?

Y

N

END

# FIG. 13

```
import torch
import torch.nn as nn

class ImprovedLSTMModelWithDropout(nn.Module):
    def __init__(self, input_size, hidden_size,
                 num_layers, output_size, dropout=0.6):
        super(ImprovedLSTMModelWithDropout, self).__init__()
        self.hidden_size = hidden_size
        self.num_layers = num_layers

        self.lstm = nn.LSTM(input_size, hidden_size, num_layers,
                 batch_first=True, dropout=dropout)

        self.fc1 = nn.Linear(hidden_size, hidden_size // 2)

        self.dropout = nn.Dropout(dropout)

        self.fc2 = nn.Linear(hidden_size // 2, output_size)

    def forward(self, x):
        out, _ = self.lstm(x)
        out = out[:, -1, :]
        out = self.fc1(out)
        out = self.dropout(out)
        out = self.fc2(out)
        return out
```

Ln: 11   Col: 43

# FIG. 14

```
def forward(self, x):
    h0 = torch.zeros(self.num_layers, x.size(0), self.hidden_size).to(x.device)
    c0 = torch.zeros(self.num_layers, x.size(0), self.hidden_size).to(x.device)

    out, _ = self.lstm(x, (h0, c0))

    out = self.fc1(out[:, -1, :])

    out = torch.relu(out)

    out = self.dropout(out)

    out = self.fc2(out)

    return out
```

Ln: 2   Col: 6

# FIG. 15

```
class EarlyStopping:
    def __init__(self, patience=15, delta=0.0005):
        self.patience = patience
        self.delta = delta
        self.counter = 0
        self.best_score = None
        self.early_stop = False
        self.val_loss_min = np.inf

    def __call__(self, val_loss, model):

        score = -val_loss


        if self.best_score is None:
            self.best_score = score
            self.save_checkpoint(val_loss, model)
        elif score < self.best_score + self.delta:

            self.counter += 1
            if self.counter >= self.patience:
                self.early_stop = True
        else:

            self.best_score = score
            self.save_checkpoint(val_loss, model)
            self.counter = 0

    def save_checkpoint(self, val_loss, model):

        torch.save(model.state_dict(), 'checkpoint.pt')
        self.val_loss_min = val_loss
```

Ln: 1   Col: 0

# FIG. 16

```
def train_model(model, X, y, num_epochs=100, learning_rate=0.0001):
    criterion = nn.MSELoss()

    optimizer = torch.optim.Adam(model.parameters(), lr=learning_rate)

    early_stopping = EarlyStopping(patience=15, delta=0.0005)

    for epoch in tqdm(range(num_epochs), desc="Training Progress"):
        model.train()
        outputs = model(X)

        optimizer.zero_grad()
        loss = criterion(outputs, y)
        loss.backward()
        optimizer.step()

        val_loss = loss.item()
        early_stopping(val_loss, model)

        if early_stopping.early_stop:
            print("\nEarly stopping")
            break

    print("Training completed")
```

Ln: 15   Col: 25

# FIG. 17

```
def evaluate_new_data(file_path, model, data_mean, data_std, device):
    required_columns = [
                        'p_leftCurrentGaitCycleRate',
                        'p_leftCurrentGRF',
                        'p_rightCurrentGaitCycleRate',
                        'p_rightCurrentGRF'
                        ]

    new_data = pd.read_csv(file_path)
    new_data = new_data[required_columns]

    new_data['p_leftCurrentGaitCycleRate'] = W
    new_data['p_leftCurrentGaitCycleRate'].astype(int)

    new_data['p_rightCurrentGaitCycleRate'] = W
    new_data['p_rightCurrentGaitCycleRate'].astype(int)

    new_data_normalized = (new_data - data_mean) / data_std
    new_data_np = new_data_normalized.to_numpy()

    X_new = new_data_np[:, [0, 2]]
    y_new = new_data_np[:, [1, 3]]

    X_new = torch.tensor(X_new, dtype=torch.float32).unsqueeze(1).to(device)
    y_new = torch.tensor(y_new, dtype=torch.float32).to(device)

    model.eval()
    with torch.no_grad():
        predictions = model(X_new)

    predictions = predictions.cpu().numpy()
    y_new = y_new.cpu().numpy()

    differences = np.abs(predictions - y_new) / y_new * 100
    mean_difference = np.mean(differences, axis=0)
    overall_mean_difference = np.mean(mean_difference)

    similarity_percentage = 100 - overall_mean_difference

    if 0 <= similarity_percentage <= 20:
        fac_grade = "FAC 5"
    elif 21 <= similarity_percentage <= 40:
        fac_grade = "FAC 1"
    elif 41 <= similarity_percentage <= 60:
        fac_grade = "FAC 2"
    elif 61 <= similarity_percentage <= 70:
        fac_grade = "FAC 3"
    elif 71 <= similarity_percentage <= 80:
        fac_grade = "FAC 4"
    elif 81 <= similarity_percentage <= 100:
        fac_grade = "FAC 5"
    else:
        fac_grade = "FAC Unknown"

    return similarity_percentage, fac_grade
```

Ln: 58   Col: 0

# FIG. 18

```
required_columns = ['p_leftCurrentGaitCycleRate', 'p_leftCurrentGRF', 'p_rightCu
file_paths = ['Data/patientTrainingResultInfoDB_guma_OG.csv',
              'Data/patientTrainingResultInfoDB_SJ Kim_OG.csv',
              'Data/patientTrainingResultInfoDB_JHCHO_OG.csv']

new_data_path = 'Data/patientTrainingResultInfoDB_guma_OG.csv'
eval_data_path = 'Data/patientTrainingResultInfoDB_PaulLee_OG.csv'
model_save_path = 'checkpoint.pt'

combined_data = load_and_combine_csv(file_paths, required_columns)
X, y, data_mean, data_std = preprocess_data(combined_data)

torch.cuda.empty_cache()
device = torch.device("cuda" if torch.cuda.is_available() else "cpu")
X = X.to(device)
y = y.to(device)

input_size = 2
hidden_size = 128
num_layers = 3
output_size = 2
dropout = 0.6

model = ImprovedLSTMModelWithDropout(input_size,
                                     hidden_size,
                                     num_layers,
                                     output_size,
                                     dropout).to(device)

train_model(model, X, y, num_epochs=100, learning_rate=0.0001)

similarity_percentage, fac_grade = evaluate_new_data(eval_data_path,
                                                     model,
                                                     data_mean,
                                                     data_std,
                                                     device)

print(f'MODEL SIMILARITY (%): {similarity_percentage}')
print(f'FAC GRADE: {fac_grade}')
```

Ln: 33  Col: 30

# FIG. 19

```
# DATA INPUT & DATAFRAME CREATION
csv_directory = 'train_data_csv'
all_dataframes = []

for filename in os.listdir(csv_directory):
    if filename.endswith('.csv'):
        file_path = os.path.join(csv_directory, filename)
        df = pd.read_csv(file_path)
        all_dataframes.append(df)
combined_dataframe = pd.concat(all_dataframes, ignore_index=True)

# DATA PREPROCESSING
    'p_patientGender', 'p_weight', 'p_footSize', 'p_patientBirthYear', 'p_fac',
    'p_trainingCurrentCadance', 'p_StrideLength', 'p_StrideHeight',
    'p_initialContactAngle', 'p_toeOffAngle',
    'p_leftCurrentGRF', 'p_rightCurrentGRF',
    'p_leftCurrentGaitCycleRate', 'p_rightCurrentGaitCycleRate'
]

combined_dataframe_relevant = combined_dataframe[relevant_columns].copy()

combined_dataframe_relevant['p_patientGender'] = (
    combined_dataframe_relevant['p_patientGender'].map({'Male': 0, 'Female': 1})
)

combined_dataframe_relevant.fillna(combined_dataframe_relevant.mean(), inplace=True)

X = torch.tensor(combined_dataframe_relevant.values, dtype=torch.float32)

mean_target = combined_dataframe_relevant[['p_trainingCurrentCadance',
                                            'p_StrideLength',
                                            'p_StrideHeight',
                                            'p_initialContactAngle',
                                            'p_toeOffAngle']].mean().values

Y = torch.tensor([mean_target] * len(X), dtype=torch.float32)
```

Ln: 85  Col: 2

# FIG. 20

```
# MOODEL DEFINITION

class TransformerModel(nn.Module):
    def __init__(self, input_dim, output_dim, num_heads=1,
                 num_layers=2, hidden_dim=128):
        super(TransformerModel, self).__init__()
        self.encoder_layer = (
            nn.TransformerEncoderLayer(
                                        d_model=input_dim,
                                        nhead=num_heads,
                                        dim_feedforward=hidden_dim
                                        )
            )
        self.transformer_encoder = nn.TransformerEncoder(self.encoder_layer,
                                                    num_layers=num_layers)
        self.fc = nn.Linear(input_dim, output_dim)

    def forward(self, x):
        x = self.transformer_encoder(x.unsqueeze(1))
        x = self.fc(x.squeeze(1))
        return x

# TRAINING SETTINGS
input_dim = X.shape[1]
output_dim = 9
batch_size = 64
learning_rate = 0.001
epochs = 100

dataset = TensorDataset(X, Y)
train_loader = DataLoader(dataset, batch_size=batch_size, shuffle=True)

model = TransformerModel(input_dim, output_dim)
criterion = nn.MSELoss()
optimizer = optim.Adam(model.parameters(), lr=learning_rate)
```

Ln: 66  Col: 14

# FIG. 21

```
# MODEL TRAINING
for epoch in range(epochs):
    model.train()
    running_loss = 0.0

    for inputs, targets in train_loader:
        optimizer.zero_grad()
        outputs = model(inputs)
        loss = criterion(outputs, targets)
        loss.backward()
        optimizer.Stride()

        running_loss += loss.item()

    print(
        f"Epoch [{epoch+1}/{epochs}], "
        f"Training Loss: {running_loss/len(train_loader):.4f}"
    )

# PREDICTING TEST DATA

test_data = pd.read_csv('test.csv')
test_data_relevant = test_data[relevant_columns].copy()
test_data_relevant['p_patientGender'] = (
    test_data_relevant['p_patientGender'].map({'Male': 0, 'Female': 1}))

test_data_relevant.fillna(test_data_relevant.mean(), inplace=True)

X_test = torch.tensor(test_data_relevant.values, dtype=torch.float32)

model.eval()
with torch.no_grad():
    prediction = model(X_test).mean(dim=0).numpy()


predicted_df = pd.DataFrame(
    [prediction],
    columns=['p_trainingCurrentCadance',
             'p_StrideLength',
             'p_StrideHeight',
             'p_initialContactAngle',
             'p_toeOffAngle']
)
print(predicted_df)
```

Ln: 128   Col: 20

EP 4 759 288 A1

# FIG. 22

**Left panel:**

Step Parameter :  ✕

Para 1    Para 2    Para 3    Kinematic 1    Kinematic 2

Cadence (4 ~ 70)    30 tempo

✓    LEFT    RIGHT    ✓

Step Length (10 ~ 55)    30 cm / 30 cm

Step Height (0 ~ 15)    10 cm / 10 cm

Initial Contact Angle (0 ~ 20)    18 ° / 18 °

Toe off Angle (-5 ~ -50)    - 23 ° / - 23 °

Ground Reaction Force (50 ~ 200)    200 % /200 %

Slope Angle (5, 10, 15, 20)    5 °

Stair Height (7, 12, 17)    7 cm

BWS (15 ~ 85)    30 %

**Right panel:**

Step Parameter :  ✕

Para 1    Para 2    Para 3    Kinematic 1    Kinematic 2

Gait Cycle Feedback ✓    Voice ✓    Target Point (%)

Left    Right    Auto    IC    MS    TO    0

Section Repeat ✓    Delay (Sec)

Left    Right    Auto    0    1    2    3

Start Point    0 %    End Point    0 %

Muscle Data

FES 1  ● L / R    FES 2  ● L / R    EMG  ● L / R

Up(%)    0    0    0

Down(%)    0    0    0

Graph

FIG. 23

TRAINING RESULT
(AVERAGE PLANTAR PRESSURE DISTRIBUTION)

# FIG. 24

```
import os
import pandas as pd
import torch
import torch.nn as nn
import torch.optim as optim
from torch.utils.data import Dataset, DataLoader


class GaitCycleDataset(Dataset):
    def __init__(self, folder_path):
        all_files = [os.path.join(folder_path, f)
                        for f in os.listdir(folder_path) if f.endswith('.csv')]
        if not all_files:
            raise ValueError
        dataframes = [pd.read_csv(file) for file in all_files]
        self.data = pd.concat(dataframes, ignore_index=True)
        self.data = self.data.fillna(0)
        self.features = self.data[['p_weight', 'p_age', 'p_height',
                                    'p_leftCurrentGRF', 'p_rightCurrentGRF',
                                    'p_bwsCurrentSupport', 'p_bodytilting',
                                    'p_kinectLeftAnkleAngle',
                                    'p_kinectRightAnkleAngle',
                                    'p_heartrate', 'p_spO2',
                                    'p_leftCurrentGaitCycleRate',
                                    'p_rightCurrentGaitCycleRate',
                                    'p_trainingCurrentCadance']]
        self.targets = self.data[['p_stepLength', 'p_stepHeight',
                                    'p_initialContactAngle', 'p_toeOffAngle']]
        self.features = ((self.features - self.features.mean()) /
                            (self.features.std() + 1e-8))
        self.targets = ((self.targets - self.targets.mean()) /
                            (self.targets.std() + 1e-8))

    def __len__(self):
        return len(self.features)

    def __getitem__(self, idx):
        x = torch.tensor(self.features.iloc[idx].values, dtype=torch.float32)
        y = torch.tensor(self.targets.iloc[idx].values, dtype=torch.float32)
        return x, y
```

Ln: 32  Col: 53

# FIG. 25

```
class GaitCycleModel(nn.Module):
    def __init__(self, input_size, hidden_size, output_size):
        super(GaitCycleModel, self).__init__()
        self.fc1 = nn.Linear(input_size, hidden_size)
        self.fc2 = nn.Linear(hidden_size, hidden_size)
        self.fc3 = nn.Linear(hidden_size, output_size)
        self.relu = nn.ReLU()

    def forward(self, x):
        x = self.relu(self.fc1(x))
        x = self.relu(self.fc2(x))
        x = self.fc3(x)
        return x
```

Ln: 53   Col: 0

## FIG. 26

```
def generate_feedback(inputs, outputs):
    feedback = []
    left_gait_cycle = inputs[11]
    right_gait_cycle = inputs[12]
    cadence = inputs[13]
    if 50 <= left_gait_cycle <= 70 and inputs[3] < inputs[4] * 0.9:
        feedback.append("Press your left foot more")
    if 50 <= right_gait_cycle <= 70 and inputs[4] < inputs[3] * 0.9:
        feedback.append("Press your right foot more")
    if inputs[6] > 10:
        feedback.append("Keep your upper body upright")
    if inputs[3:5].mean() < outputs[0]:
        feedback.append("Stay strong")
    if inputs[9] > 120:
        feedback.append("Your heart rate is increasing. Take a break.")
    return feedback
```

Ln: 53   Col: 0

FIG. 27

```
def process_realtime_data(row, model):
    inputs = row.values
    inputs_tensor = torch.tensor(inputs, dtype=torch.float32).unsqueeze(0)
    outputs = model(inputs_tensor).detach().numpy()[0]
    feedback = generate_feedback(inputs, outputs)
    return feedback, outputs
```
Ln: 53    Col: 0

# FIG. 28

```
def train_model(model, dataloader, criterion, optimizer, epochs=10):
    model.train()
    for epoch in range(epochs):
        total_loss = 0.0
        for inputs, targets in dataloader:
            optimizer.zero_grad()
            outputs = model(inputs)
            loss = criterion(outputs, targets)
            loss.backward()
            optimizer.step()
            total_loss += loss.item()
        print(f"Epoch {epoch+1}/{epochs}, Loss: {total_loss:.4f}")
```

Ln: 53   Col: 0

# FIG. 29

```
def evaluate_model(model, dataloader):
    model.eval()
    with torch.no_grad():
        for inputs, targets in dataloader:
            outputs = model(inputs)
            feedback = generate_feedback(Inputs[0].numpy(),
                                        outputs[0].numpy())
            print("Feedback:", feedback)
            print("Predicted Parameters:", outputs[0].numpy())
            print("Actual Parameters:", targets[0].numpy())
```

Ln: 104   Col: 41

# FIG. 30

```
if __name__ == "__main__":
    folder_path = r"C:\Users\USER\Desktop\Model 3\train_data"
    dataset = GaitCycleDataset(folder_path)
    dataloader = DataLoader(dataset, batch_size=16, shuffle=True)
    input_size = len(dataset.features.columns)
    hidden_size = 64
    output_size = len(dataset.targets.columns)
    model = GaitCycleModel(input_size, hidden_size, output_size)
    criterion = nn.MSELoss()
    optimizer = optim.Adam(model.parameters(), lr=0.001)
    print("Training Model...")
    train_model(model, dataloader, criterion, optimizer, epochs=30)
    print("\nEvaluating Model...")
    evaluate_model(model, dataloader)
    print("\nProcessing Real-time Data...")
    for _, row in dataset.features.iterrows():
        feedback, outputs = process_realtime_data(row, model)
        print(f"Feedback: {feedback}")
        print(f"Predicted Parameters: {outputs}")
    torch.save(model.state_dict(), "gait_cycle_model.pth")
    print("Model Saved!")
```

Ln: 99    Col: 25

# FIG. 31

```
device = torch.device("cuda" if torch.cuda.is_available() else "cpu")

input_size = 15, hidden_size = 64, num_layers = 2, seq_length = 20
batch_size = 32, learning_rate = 0.001, epochs = 20

folder_path = 'training_dataset'
all_files = [os.path.join(folder_path, f) for f in os.listdir(folder_path)
             if f.endswith('.csv')]
data_list = []

for file in all_files:
    data = pd.read_csv(file, index_col='p_rowid')
    data_list.append(data)

data = pd.concat(data_list, ignore_index=True)

columns_to_use_updated = {
    'p_trainingCompletionNumber': 'Gait Cycle Identifier',
    'p_leftCurrentGRF': 'Left GRF','p_rightCurrentGRF': 'Right GRF',
    'p_bwsCurrentSupport': 'BWS','p_trainingCurrentCadance': 'Cadence',
    'p_leftCurrentGaitCycleRate': 'Left Gait Cycle',
    'p_rightCurrentGaitCycleRate': 'Right Gait Cycle',
    'p_stepLength': 'Step Length', 'p_stepHeight': 'Step Height',
    'p_initialContactAngle': 'Initial Contact Angle',
    'p_toeOffAngle': 'Toe Off Angle', 'p_footSize': 'Foot Size',
    'p_weight': 'Weight', 'p_age': 'Age','p_height': 'Height',
    'p_fac': 'FAC Grade'
}

existing_columns = [col for col in columns_to_use_updated.keys()
                    if col in data.columns]
filtered_data = data[existing_columns].rename(columns=columns_to_use_updated)

train_data, test_data = train_test_split(filtered_data, test_size=0.3,
                                         random_state=42)

train_grouped = train_data.groupby("Gait Cycle Identifier")
test_grouped = test_data.groupby("Gait Cycle Identifier")
```

Ln: 13  Col: 40

# FIG. 32

```
class LSTMAutoencoder(nn.Module):
    def __init__(self, input_size, hidden_size, num_layers):
        super(LSTMAutoencoder, self).__init__()
        self.encoder = nn.LSTM(input_size, hidden_size, num_layers, batch_first=True)
        self.decoder = nn.LSTM(hidden_size, input_size, num_layers, batch_first=True)

    def forward(self, x):
        encoded, _ = self.encoder(x)
        decoded, _ = self.decoder(encoded)
        return decoded

class Seq2SeqLSTM(nn.Module):
    def __init__(self, input_size, hidden_size, num_layers):
        super(Seq2SeqLSTM, self).__init__()
        self.encoder = nn.LSTM(input_size, hidden_size, num_layers, batch_first=True)
        self.decoder = nn.LSTM(hidden_size, hidden_size, num_layers, batch_first=True)
        self.output_layer = nn.Linear(hidden_size, input_size)

    def forward(self, x):
        encoded, (hidden, cell) = self.encoder(x)
        decoded, _ = self.decoder(encoded, (hidden, cell))
        output = self.output_layer(decoded)
        return output

anomaly_model = LSTMAutoencoder(input_size, hidden_size, num_layers).to(device)
prediction_model = Seq2SeqLSTM(input_size, hidden_size, num_layers).to(device)

criterion = nn.MSELoss()
anomaly_optimizer = optim.Adam(anomaly_model.parameters(), lr=learning_rate)
prediction_optimizer = optim.Adam(prediction_model.parameters(), lr=learning_rate)
```

Ln: 62    Col: 0

FIG. 33

```
def train_anomaly_model(grouped_data, epochs):
    print("Training Anomaly Detection Model...")
    for epoch in range(epochs):
        for _, group in grouped_data:
            group = group[['Left GRF', 'Right GRF', 'BWS', 'Cadence',
                          'Left Gait Cycle','Right Gait Cycle', 'Step Length',
                          'Step Height', 'Initial Contact Angle','Toe Off Angle',
                          'Foot Size', 'Weight', 'Age', 'Height', 'FAC Grade']].values
            group_tensor = torch.tensor(group, dtype=torch.float32).unsqueeze(0).to(device)

            outputs = anomaly_model(group_tensor)
            loss = criterion(outputs, group_tensor)

            anomaly_optimizer.zero_grad()
            loss.backward()
            anomaly_optimizer.step()

        if (epoch + 1) % 5 == 0:
            print(f"Epoch [{epoch+1}/{epochs}], Loss: {loss.item():.4f}")
```

Ln: 99  Col: 0

# FIG. 34

```
def train_prediction_model(grouped_data, epochs):
    print("\nTraining Trend Prediction Model...")
    for epoch in range(epochs):
        for _, group in grouped_data:
            group = group[['Left GRF', 'Right GRF', 'BWS', 'Cadence', 'Left Gait Cycle',
                           'Right Gait Cycle', 'Step Length', 'Step Height',
                           'Initial Contact Angle','Toe Off Angle', 'Foot Size', 'Weight',
                           'Age', 'Height', 'FAC Grade']].values
            group_tensor = torch.tensor(group, dtype=torch.float32).unsqueeze(0).to(device)

            outputs = prediction_model(group_tensor)
            loss = criterion(outputs, group_tensor)

            prediction_optimizer.zero_grad()
            loss.backward()
            prediction_optimizer.step()

        if (epoch + 1) % 5 == 0:
            print(f"Epoch [{epoch+1}/{epochs}], Loss: {loss.item():.4f}")
```

Ln: 122  Col: 0

# FIG. 35

```
def evaluate_anomaly_model(grouped_data):
    print("\nEvaluating Anomaly Detection Model...")
    total_loss = 0
    for _, group in grouped_data:
        group = group[['Left GRF', 'Right GRF', 'BWS', 'Cadence', 'Left Gait Cycle',
                       'Right Gait Cycle', 'Step Length', 'Step Height', 'Initial Contact Angle',
                       'Toe Off Angle', 'Foot Size', 'Weight', 'Age', 'Height',
                       'FAC Grade']].values
        group_tensor = torch.tensor(group, dtype=torch.float32).unsqueeze(0).to(device)

        with torch.no_grad():
            outputs = anomaly_model(group_tensor)
            loss = criterion(outputs, group_tensor)
            total_loss += loss.item()

    avg_loss = total_loss / len(grouped_data)
    print(f"Average Reconstruction Loss: {avg_loss:.4f}")
```

Ln: 135  Col: 23

FIG. 36

```
DEFINITION visualize_analyze_and_suggest_results(test_grouped):
    PRINT "Generating Visualization, Analysis, and Suggestions..."
    INITIALIZE analysis_results = []
    INITIALIZE overall_score = 0
    CALCULATE num_cycles = len(test_grouped)

    FOR each (key, group) IN test_grouped:
        EXTRACT relevant features FROM group INTO group_tensor
        PREDICT predictions USING prediction_model(group_tensor)
        CALCULATE differences BETWEEN group and predictions
        COMPUTE mean_diff, std_diff, mse, mae FROM differences
        COMPUTE cycle_score BASED ON mse AND ADD TO overall_score

        APPEND metrics (mean_diff, std_diff, mse, mae, cycle_score) TO analysis_results

        PLOT "Actual vs Predicted" graph FOR current group

        INITIALIZE suggestions = []
        FOR each feature IN monitored_features:
            IF deviation of feature EXCEEDS threshold:
                ADD suggestion FOR feature TO suggestions

        IF suggestions EXIST:
            PRINT suggestions FOR current gate cycle

    CALCULATE overall_score AS overall_score / num_cycles
    PRINT "Overall Performance Score:", overall_score
    PRINT "Detailed Analysis Results:", analysis_results
END DEFINITION
```

Ln: 20   Col: 54

## INTERNATIONAL SEARCH REPORT

| | International application No. |
|---|---|
| | **PCT/KR2025/099186** |

### A. CLASSIFICATION OF SUBJECT MATTER

**A61H 1/02**(2006.01)i; **A61B 5/11**(2006.01)i; **G16H 10/60**(2018.01)i; **G16H 20/30**(2018.01)i; **G16H 50/20**(2018.01)i

According to International Patent Classification (IPC) or to both national classification and IPC

### B. FIELDS SEARCHED

Minimum documentation searched (classification system followed by classification symbols)

A61H 1/02(2006.01); A61H 3/00(2006.01); B25J 9/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 페달(pedal), 링크(link), 보행 운동 장치(gait training apparatus), 훈련 계획(training plan), 맞춤형 훈련(personalized training), 모니터링(monitoring), 피드백(feedback), 평가(evaluation), 재활(rehabilitation)

### C. DOCUMENTS CONSIDERED TO BE RELEVANT

| Category* | Citation of document, with indication, where appropriate, of the relevant passages | Relevant to claim No. |
|---|---|---|
| Y | KR 10-2024-0013925 A (HIWIN TECHNOLOGIES CORP.) 31 January 2024 (2024-01-31)<br>See paragraphs [0014], [0021] and [0036]-[0038]; and figures 2-2h. | 1-20 |
| Y | JP 2021-003490 A (TOYOTA MOTOR CORP.) 14 January 2021 (2021-01-14)<br>See paragraphs [0007], [0036], [0054]-[0068], [0083]-[0102], [0122]-[0145] and [0151]-[0201]; claims 1 and 8; and figure 2. | 1-20 |
| Y | JP 2021-003541 A (TOYOTA MOTOR CORP.) 14 January 2021 (2021-01-14)<br>See paragraphs [0190]-[0191] and [0193]. | 1-10,13-14 |
| Y | JP 2011-019669 A (SUNCALL ENGINEERING K.K.) 03 February 2011 (2011-02-03)<br>See paragraphs [0049] and [0052]; and figure 1. | 8-9,18 |
| A | KR 10-2024-0009528 A (SAMSUNG ELECTRONICS CO., LTD.) 22 January 2024 (2024-01-22)<br>See paragraphs [0024]-[0056]; and figures 2-4. | 1-20 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **02 May 2025** | **02 May 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2025/099186**

| Patent document cited in search report | | | Publication date (day/month/year) | Patent family member(s) | | | Publication date (day/month/year) |
|---|---|---|---|---|---|---|---|
| KR 10-2024-0013925 | | A | 31 January 2024 | KR | 10-2678113 | B1 | 25 June 2024 |
| JP 2021-003490 | | A | 14 January 2021 | CN | 112137839 | A | 29 December 2020 |
| | | | | CN | 112137839 | B | 01 August 2023 |
| | | | | EP | 3758011 | A1 | 30 December 2020 |
| | | | | JP | 7243486 | B2 | 22 March 2023 |
| | | | | US | 11839465 | B2 | 12 December 2023 |
| | | | | US | 2020-0405189 | A1 | 31 December 2020 |
| JP 2021-003541 | | A | 14 January 2021 | CN | 112137835 | A | 29 December 2020 |
| | | | | CN | 112137835 | B | 14 April 2023 |
| | | | | JP | 7326927 | B2 | 16 August 2023 |
| | | | | US | 12093815 | B2 | 17 September 2024 |
| | | | | US | 2020-0410339 | A1 | 31 December 2020 |
| JP 2011-019669 | | A | 03 February 2011 | JP | 5386253 | B2 | 15 January 2014 |
| KR 10-2024-0009528 | | A | 22 January 2024 | CN | 118891134 | A | 01 November 2024 |
| | | | | EP | 4438239 | A1 | 02 October 2024 |
| | | | | KR | 10-2023-0101683 | A | 06 July 2023 |
| | | | | KR | 10-2023-0103949 | A | 07 July 2023 |
| | | | | KR | 10-2025-0016419 | A | 03 February 2025 |
| | | | | KR | 10-2625749 | B1 | 17 January 2024 |
| | | | | KR | 10-2760203 | B1 | 03 February 2025 |
| | | | | US | 2024-0350036 | A1 | 24 October 2024 |
| | | | | WO | 2023-128619 | A1 | 06 July 2023 |

Form PCT/ISA/210 (patent family annex) (July 2022)